# EUROPEAN PATENT SPECIFICATION

(11) **EP 2 177 163 B1**
(45) Date of publication and mention of the grant of the patent: **07.09.2011**
(21) Application number: 10152639.0
(22) Date of filing: 01.07.2005
(51) Int. Cl.: A61B 6/00, A61B 5/00, G01N 21/64, G02B 7/32, G06T 5/00

(54) **Projection of subsurface structure onto an object's surface**
Projektion von Unteroberflächenstrukturen auf eine Objektoberfläche
Projection d'une structure subsurfacique sur la surface de l'objet

(43) Date of publication of application: 21.04.2010
(62) Divisional of application: 05764289.4
(73) Proprietor: Luminetx Corporation, Memphis, TN 38104 (US)
(72) Inventor: Zeman, Herbert, Memphis, TN 38103 (US); Lovhoiden, Gunnar, Barlett, TN Tennessee 38133 (US); Vrancken, Carlos, Solihull, West Midlands, B91 2LE (GB); Snodgrass, John, Denton, TX Texas 76201 (US); Delong, James, Denton, TX Texas 76201 (US)
(74) Representative: Indahl, Peter Jensen

(56) References cited:
- WO-A-01/54393
- DE-U1- 20 115 964
- David Tschumperlé: "Thèse - PDE's based regularization of multivalued images and applications" 13 December 2002 (2002-12-13), Université de Nice - Sophia Antipolis , XP002572052 * page 111, paragraph 2 - page 117, paragraph 3 * * figure 4.6 *

## Description

### TECHNICAL FIELD

The present invention is generally directed to generation of diffuse infrared light. More particularly, the invention is directed to a system for illuminating an object with diffuse infrared light, producing a video image of buried structure beneath the surface of the object based on reflected infrared light, and then projecting an image of the buried structure onto the surface of the object.

### BACKGROUND OF THE INVENTION

Some medical procedures and treatments require a medical practitioner to locate a blood vessel in a patient's arm or other appendage. This can be a difficult task, especially when the blood vessel is small and/or the vessel is under a significant deposit of subcutaneous fat or other tissue. The performance of previous imaging systems designed to aid in finding such blood vessels has been lacking.

For instance WO 01/54393 discloses an apparatus to enhance the visibility of buried structure beneath the surface of an object, which apparatus comprises an imaging device for receiving diffuse light reflected from the object and for producing an image.

Therefore, a system for enhancing the visual contrast between subcutaneous blood vessels and surrounding tissue is needed.

### BRIEF SUMMARY OF THE INVENTION

The foregoing and other needs are met by an apparatus as defined in claim 1.

In another embodiment, an apparatus is disclosed for providing diffuse light to an object. The apparatus includes an array of light-emitting sources, each source for emitting infrared light having a wavelength toward the object. A power source provides power to the array. The apparatus further includes diffusing structure which provides various levels of diffusion to the infrared light emitted from the array. The diffusing structure includes a first diffusing layer which is disposed adjacent to the array. The first diffusion layer provides a first level of diffusion to the light emitted by the array. A second diffusing layer is spaced apart from the first diffusing layer and provides a second level of diffusion to the light emitted by the array. A polarizer is included to polarize the light emitted by the array.

In yet another embodiment, an apparatus is disclosed which provides diffuse light to an object. The apparatus includes a light source for emitting infrared light toward the object. A first diffusing layer having a first diffusing plane intercepts light from the light source and provides a first amount of diffusion to the infrared light emitted by the light source. The apparatus includes a video imaging device for receiving light reflected from the object. The video imaging device operates to provide a video image of the object based on the reflected light.

In yet another embodiment, an apparatus is disclosed for providing diffuse light to an object. Groups of light-emitting diodes (LEDs) are arranged in a select pattern which define an LED plane. Each LED has an emitting surface for emitting infrared light towards the object and an electrical input for providing an electrical signal to the LED. The apparatus includes a control circuit which provides control signals to activate one or more LEDs in a select group of LEDs. A diffusing structure is positioned to intercept and diffuse the infrared light emitted from one or more of the LEDs.

Using the invention described herein, subcutaneous blood vessels that are difficult or impossible to see under white light or under non-diffuse infrared light can be easily seen in a video image, where the subcutaneous blood vessels appear as dark lines against a lighter background of surrounding flesh.

Two mechanisms are described for keeping the image of the buried structure, as seen by the imaging device, in focus with a proper lens-to-subject distance. A first embodiment of this mechanism uses a pair of laser pointers directed toward the object from different angles, such that the two laser pointers only converge to the same spot when the target is at the proper lens-to-subject distance from the imaging device. A second embodiment of this mechanism adds a recognizable pattern, such as a text border, to the projected image such that the projected recognizable pattern will only be in focus on the surface of the target object when the target is at the proper lens-to-subject distance from the projector, thereby causing the target to also be at the proper lens-to subject distance from the imaging device.

Image processing is disclosed that removes undesired small artifacts, such as surface hair and other features, from the viewed image of buried structure prior to projection onto the surface of the object.

A calibration procedure is described wherein the projector projects a green target pattern onto a fluorescent screen, which converts the projected green target pattern into deep red light that is visible by the infrared imaging device. A computer program records the position of the viewed pattern and calculates calibration coefficients to be used in a bi-linear transformation to correct magnification, rotation, and translation misalignment between the imaging device and the projector.

### TECHNICAL ASPECTS OF THE INVENTION

From a technical point of view, the present invention addresses a situation, wherein some medical procedures and treatments require a medical practitioner to locate a blood vessel in a patient's arm or other appendage. In the prior art, this could be a difficult task, especially when the blood vessel lies under a significant deposit of subcutaneous fat. The performance of previous imaging systems designed to aid in finding such blood vessels has been lacking. It is therefore the technical problem underlying the present invention to provide an apparatus and method for enhancing the visual contrast between subcutaneous blood vessels and surrounding tissue.

This problem is solved by an apparatus to enhance the visibility of a buried structure beneath the surface of an object. The medical device comprises an imaging device for receiving diffuse light reflected from an object and for producing an image and a video projector for projecting a visible light image of the buried structure onto the surface of the obj ect.

The technical idea underlying the invention is a conceptual change by imaging the underlying structure and by using a first laser having a first emitted beam and a second laser having a second emitted beam, said first emitted beam and said second emitted beam being non-parallel with respect to each other and said first and said second emitted beams intersecting at a desired target distance from said apparatus at which, when the intersection of said first and said second emitted beams is upon the surface of the object, said buried structure is in focus to said imaging device. As a result, the difficult task of locating a blood vessel in a patient's arm or other appendage is much easier because the blood vessel becomes visible as an image projected on the skin.

Preferably, the apparatus also comprises video projector means for displaying the underlying structure by projecting a visible light image onto the surface of the object.

According to a further aspect, the present invention relates to a method of calibrating an apparatus that enhances the visibility of buried structure beneath the surface of an object. The apparatus comprises an imaging device for receiving diffuse light within a first spectrum reflected from the object and for producing an image; and video projector means for projecting a visible light image within a second spectrum of the buried structure onto the surface of the object, said first and said second spectrums being substantially non-overlapping. The method comprises the steps of projecting a pattern of spaced-apart dots in said second spectrum onto a fluorescent screen that fluoresces thereat in said second spectrum; then observing the positions of said fluoresced pattern of dots; then using said observed positions to calculate calibration coefficients for a bi-linear transformation; then correcting a subsequently-projected image using said bi-linear transformation with said calibration coefficients.

According to a further aspect, the present invention relates to a method for enhancing the visibility of a buried structure beneath the surface of an object. The method comprises the steps of providing an imaging device for receiving diffuse light reflected from the object; producing a received image; then removing small dark artifacts from the received image while retaining larger dark objects in the received image to produce a processed image; then projecting the processed image onto the surface of the object in the visible spectrum.

Preferably, the method additionally comprises, following the production of a processed image and before projecting the processed image, the step of performing adaptive edge enhancement upon the processed image.

According to a further aspect, the present invention relates to an apparatus to enhance the visibility of buried structure beneath the surface of an object. The apparatus comprises an imaging device for receiving diffuse light reflected from the object and for producing an image; and video projector means for projecting a visible light image of the buried structure onto the surface of the object, said projected visible light image including a recognizable added pattern that is independent of said buried structure.

Preferably, the added pattern is a text border.

According to a further aspect, the present invention relates to another apparatus to enhance the visibility of buried structure beneath the surface of an object. The apparatus comprises an imaging device for receiving diffuse light reflected from the object and for producing an image; video projector means for projecting a visible light image of the buried structure onto the surface of the object; and an infrared filter interposed between said imaging device and said object.

According to yet a further aspect, the present invention relates to a third apparatus to enhance the visibility of buried structure beneath the surface of an object. The apparatus comprises an imaging device for receiving diffuse light reflected from the object and for producing an image; video projector means for projecting a visible light image of the buried structure onto the surface of the object; and an illumination source for illuminating the buried structure.

Preferably, the apparatus, in which said imaging device receives only said diffuse reflected light within a first spectrum, and said visible light image of the buried structure projected onto the surface of the object has a second spectrum that is substantially non-overlapping with said first spectrum.

In a preferred embodiment, the apparatus additionally comprises an infrared filter interposed between said imaging device and said object.

According to a further aspect, the present invention relates to a fourth apparatus to enhance the visibility of buried structure beneath the surface of an object, in which said buried structure is illuminated by ambient light. The apparatus comprises an imaging device for receiving diffuse light reflected from the object and for producing an image; video projector means for projecting a visible light image of the buried structure onto the surface of the object; and an infrared filter interposed between said imaging device and said object.

### BRIEF DESCRIPTION OF THE DRAWINGS

Further advantages of the invention will become apparent by reference to the detailed description of preferred embodiments when considered in conjunction with the drawings, which are not to scale, wherein like reference characters designate like or similar elements throughout the several drawings as follows:

FIG. 1 depicts an imaging system for viewing an object under infrared illumination according to a preferred embodiment of the invention;

FIGS. 2a and 2b are perspective views of an imaging system using diffuse infrared light according to a preferred embodiment of the invention;

FIGS. 3 and 4 are cross-sectional views of the imaging system according to a preferred embodiment of the invention;

FIG. 5 is a functional block diagram of the imaging system according to a preferred embodiment of the invention;

FIG. 6a is a perspective view of an imaging system using diffuse infrared light according to an alternative embodiment of the invention;

FIG. 6b is a cross-sectional view of the imaging system of FIG. 6a;

FIG. 7a is a perspective view of an imaging system using diffuse infrared light according to another embodiment of the invention;

FIG. 7b is a cross-sectional view of the imaging system of FIG. 7a;

FIG. 8 is an isometric view of yet another aspect of an imaging system;

FIG. 9 is a front view of a portion of the imaging system as viewed in the direction of the arrows taken along line A-A of FIG. 8;

FIG. 10 is a cross-sectional side view taken along line B-B of FIG. 9 and,

FIG. 11 is a block diagram of an imaging system.

FIG. 12 is a perspective internal view of a third version of the imaging system of the present invention.

FIG. 13 is an internal view of a fourth version of the imaging system of the present invention with some parts shown in section for purposes of explanation.

FIG. 14 is a diagrammatic view of the fourth version of the imaging system of the present invention.

FIG. 15 is an internal view of a fifth version of the imaging system of the present invention, which uses ambient lighting to illuminate the viewed object.

FIGS. 16a and 16b, taken together in sequence, are a program listing for artifact removal image processing of the received image.

FIGS. 17a, 17b, 17c, 17d, 17e, and 17f, taken together in sequence, are a program listing in the C++ programming language for artifact removal image processing of the received image.

FIG. 18 is a diagrammatic perspective view showing how a pair of laser pointers are used to position the object to be viewed.

Fig. 19 is a diagrammatic perspective view showing the calibration procedure for the imaging system of the present invention.

FIGS. 20a, 20b, and 20c are photographs of a processed image of subcutaneous blood vessels projected onto body tissue that covers the blood vessels.

FIG. 21 is a photograph of a projected image having a text border therearound.

FIG. 22 is another photograph of a projected image having a text border therearound, similar to FIG. 21 but in which the viewed object has been moved out of position, showing how the text border becomes out-of-focus to indicate that the object is not positioned properly.

FIG. 23 shows a text border image that is combined with a projected image for joint projection onto the object to ensure proper positioning.

FIG. 24 is a photograph of a processed image of subsurface veins projected onto a hand by the present invention, similar to Fig. 20 (which omits the text border) and Fig. 21 but showing how the text border becomes out of focus to indicate that the hand is not positioned properly.

FIG. 25a and FIG. 25b are computer listings showing the solution for bi-linear transformation coefficients of the calibration procedure for the imaging system of the present invention.

FIG. 26 is a program listing in the C++ programming language, which performs the run-time correction to the viewed image of the object using coefficients determined during the calibration procedure.

### DETAILED DESCRIPTION OF THE INVENTION

Skin and some other body tissues reflect infrared light in the near-infrared range of about 700 to 900 nanometers, while blood absorbs radiation in this range. Thus, in video images of body tissue taken under infrared illumination, blood vessels appear as dark lines against a lighter background of surrounding flesh. However, due to the reflective nature of subcutaneous fat, blood vessels that are disposed below significant deposits of such fat can be difficult or impossible to see when illuminated by direct light, that is, light that arrives generally from a single direction.

The inventor has determined that when an area of body tissue having a significant deposit of subcutaneous fat is imaged in near-infrared range under illumination of highly diffuse infrared light, there is significantly higher contrast between the blood vessels and surrounding flesh than when the tissue is viewed under direct infrared illumination. Although the invention should not be limited by any particular theory of operation, it appears that most of the diffuse infrared light reflected by the subcutaneous fat is directed away from the viewing direction. Thus, when highly diffuse infrared light is used to illuminate the tissue, the desired visual contrast between the blood vessels and the surrounding flesh is maintained.

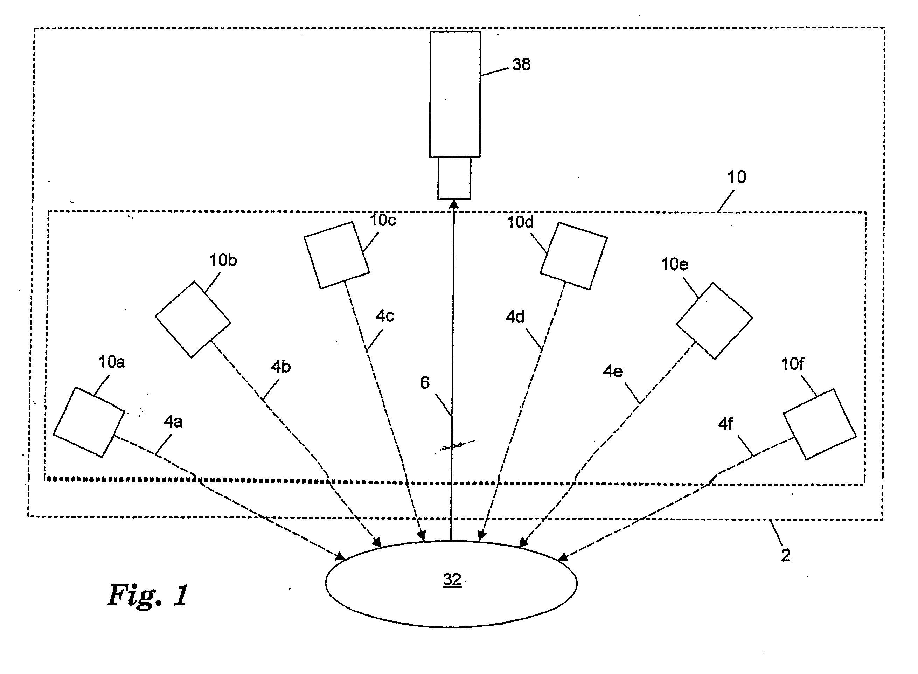

Shown in FIG. 1 is an imaging system **2** for illuminating an object **32,** such as body tissue, with highly diffuse infrared light, and for producing a video image of the object **32** based upon infrared light reflected from the object **32.** As described in detail herein, when the object **32** is body tissue, blood vessels that are disposed below subcutaneous fat in the tissue may be clearly seen in a video image produced by the system **2.**

The imaging system **2** includes an illumination system **10** that illuminates the object **32** with infrared light from multiple different illumination directions. The system **10** includes multiple infrared light providers **10a-10f,** each providing infrared light to the object **32** from a different illumination direction. The directions of arrival of the infrared light from each light provider **10a-10f** are represented in FIG. 1 by the rays **4a-4f.** As shown in FIG. 1, the directions of arrival of the infrared light ranges from perpendicular or near perpendicular to the surface of the object **32,** to parallel or near parallel to the surface of the object **32.** Since the infrared illumination arrives at the object **32** from such a wide range of illumination directions, the infrared illumination is highly diffuse.

As described in greater detail hereinafter, the light providers **10a-10f** are preferably light reflecting surfaces that direct light from a single light source toward the object **32.** In other embodiments, the light providers **10a-10f** are individual light sources, or combinations of light sources and reflectors.

The imaging system **2** also includes an imaging device **38,** such as a video camera, for viewing the object **32.** The imaging device **38** views the object **32** from a viewing direction which is represented in FIG. 1 by the arrow **6.** The imaging device **38** receives the diffuse infrared light reflected from the object **32,** and generates an electronic video image of the object **32** based on the reflected infrared light.

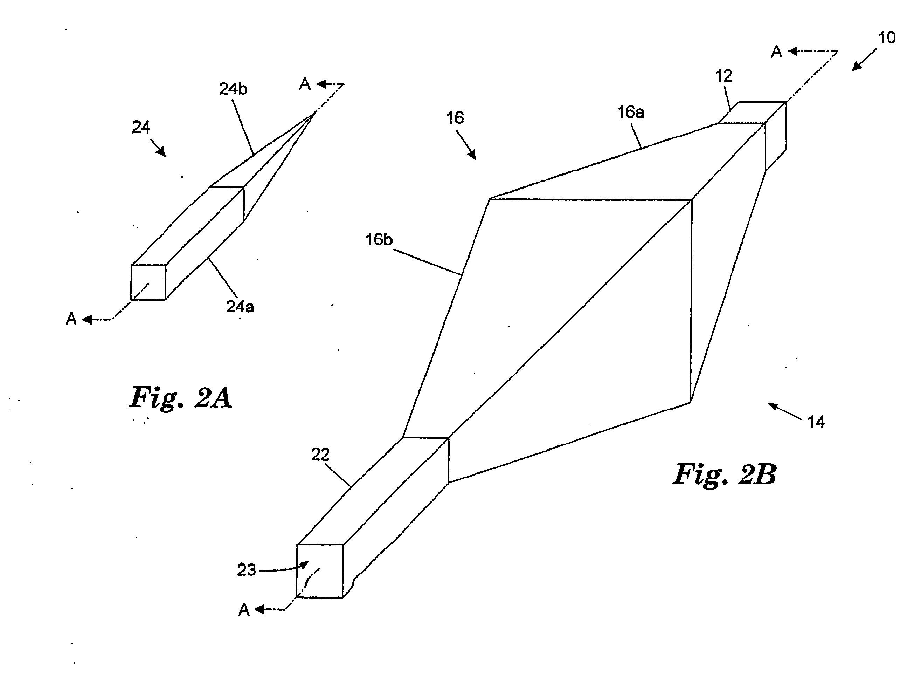

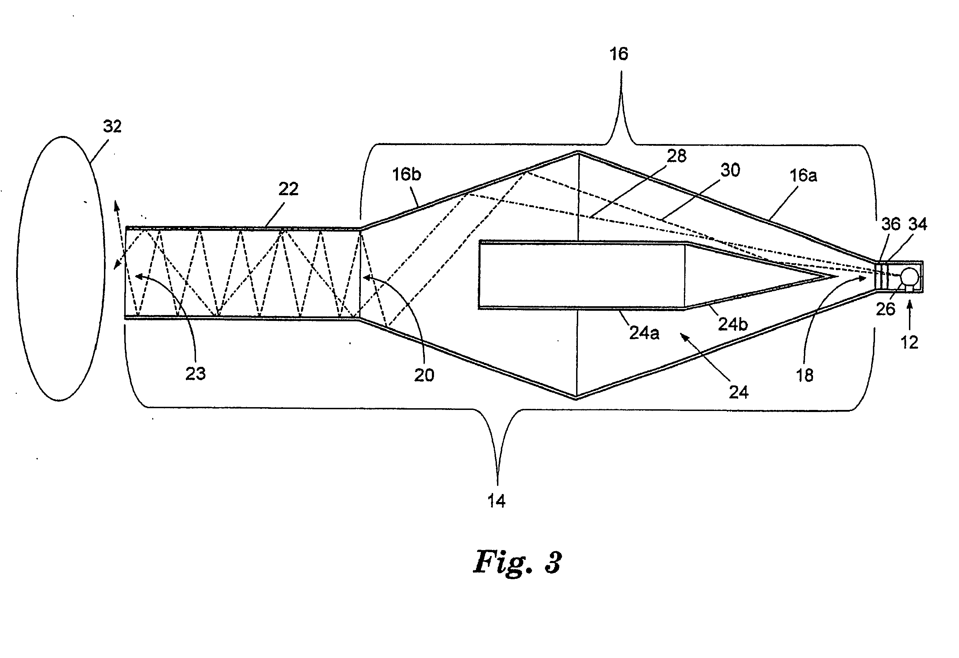

Shown in FIGS. 2a and 2b is a preferred embodiment of the illumination system 10. FIG. 3 depicts a cross-sectional view of the system **10** corresponding to the section A-A as shown in FIGS. 2a-b. The system **10** preferably includes a light source **12** that emits light into one end of a light diffusing structure **14.** The light diffusing structure **14** includes an elongate outer enclosure **16** having reflective inner surfaces. Preferably, the inner surfaces of the elongate outer enclosure **16** are white in color. Alternatively, these reflective surfaces are mirrored surfaces, or a combination of white and mirrored surfaces. At the end of the light diffusing structure **14** opposite the light source **12,** is a hollow light guide **22.** As described in more detail below, the light guide **22** serves as an output aperture for the diffuse light.

The elongate outer enclosure **16** includes first and second sections **16a** and **16b,** each having a large end and a small end. Preferably, the first and second sections **16a** and **16b** are substantially pyramidal in shape, each having four trapezoidal faces. In the preferred embodiment, the four trapezoidal faces of the sections **16a** and **16b** are identical, such that each end of the sections **16a** and **16b** forms a square aperture. As shown in FIGS. 2a and 2b, the larger ends of the first and second sections **16a** and **16b** are joined together to form the enclosure **16.**

At the small end of the first section **16a** is an input aperture **18** formed by the four short sides of the four trapezoidal faces of the section **16a.** The light source **12** is preferably attached to the small end of the first section **16a** at the input aperture **18.** Thus, the light generated by the light source **12** enters the elongate enclosure **16** at the input aperture **18,** and illuminates the interior surfaces of the enclosure **16.**

At the small end of the second section **16b** is an output aperture **20** formed by the four short sides of the four trapezoidal faces of the section **16b.** Attached at the output aperture **20** is one end of the hollow light guide **22.** The light guide **22** preferably has white reflective inner surfaces similar to the inner surfaces of the enclosure **16.**

The system **10** also includes an elongate inner reflector **24** which is disposed within and preferably coaxial with the outer enclosure **16.** For clarity, the inner reflector **24** is shown in FIG. 2b removed from the outer enclosure **16.** In the preferred embodiment, the inner reflector **24** is formed from a square tubular section **24a** joined to the square base of a pyramidal section **24b.** Preferably, the pyramidal section **24b** has four sides that taper down to an apex. As shown in FIG. 3, the apex of the pyramidal section **24b** is disposed proximate the input aperture **18** of the outer enclosure **16.** The inner reflector **24** has reflective white outer surfaces similar to those of the inner surfaces of the outer enclosure 16.

The light diffusing characteristics of the structure **14** are best understood with reference to FIG. 3. Within the light source **12** is a lamp **26,** such as a quartz-halogen bulb and gold-plated reflector manufactured by Gilway and having part number L517A-G. When energized, the lamp **26** produces electromagnetic radiation in the form of white light.

For purposes of this description, the lamp **26** may be thought of as a point source radiating light in multiple directions, as represented by the exemplary rays **28** and **30.** As shown in FIG. 3, the ray **28** reflects from the inner surface of the section **16b** of the outer enclosure **16.** The ray **28** then travels through the output aperture **20,** into the light guide **22,** and, after multiple reflections from the inner surfaces of the light guide **22,** emits from the exit aperture **23.** The ray **30,** which exits the light source **12** from a different angle than the ray **28,** reflects from the inner reflector **24.** The ray **30** then reflects from the inner surface of the section **16b** of the outer enclosure **16,** and travels through the output aperture **20** and into the light guide **22.** After multiple reflections from the inner surfaces of the light guide **22,** the ray **30** also emits from the exit aperture **23,** but at a different angle than that of the ray **28.**

When an object **32** is placed near the exit aperture **23,** the rays **28** and **30** arrive at the object **32** from different angles. It will be appreciated that the light radiating from the light source **12** could be represented as an infinite number of rays which strike and reflect from the inner reflector **24** and the inner surfaces of the outer enclosure **16** from an infinite number of angles. Thus, the light emitted from the exit aperture **23** arrives at the object **32** from many different angles, and is therefore highly diffuse light. These arrival angles range from near perpendicular to near parallel with the plane of the exit aperture **23.** Since the diffusing structure **14** is three-dimensional, it will be appreciated that light also reflects from the other surfaces of the outer enclosure **16** and the inner reflector **24,** such as those that are perpendicular to the surfaces shown in FIG. 3. Therefore, the light emitted at the exit aperture **23** of the illumination system **10** is highly diffuse, appearing to be generated by many different light sources.

Due to the arrangement of the reflective inner surfaces of the outer enclosure **16** and the reflective outer surfaces of the inner reflector **24,** the diffusing structure **14** efficiently transfers the light radiated from the lamp **26** to the exit aperture **23.** Thus, a very large fraction of the light provided by the lamp **26** reaches the object **32,** and very little light energy is wasted.

As described in more detail below, the illumination system **10** can be used to provide diffuse light for medical imaging purposes. However, it will be appreciated that the scope of the invention is not limited to medical uses. The system **10** could also be used as a diffuse light source for general photographic purposes.

In a preferred embodiment of the invention, as depicted in FIG. 3, the light source **12** includes a cold mirror **34** disposed between the lamp **26** and the input aperture **18** of the outer enclosure **16.** The cold mirror **34** reflects substantially all light having wavelengths outside a selected infrared range of wavelengths. Preferably, the selected range includes wavelengths from approximately 700 to 1000 nanometers. Immediately proximate the cold mirror **34,** and disposed between the cold mirror **34** and the input aperture **18,** is an infrared transmitting filter **36** which further attenuates light having wavelengths outside the selected infrared range while transmitting light hawing wavelengths within the selected infrared range. Thus, the light that passes through the cold mirror **34** and the filter **36** into the outer enclosure **16** is infrared light having wavelengths within the selected infrared range.

It should be appreciated that there are other ways that the light source **12** could be configured to generate infrared light. For example, the light source **12** could consist of an infrared light-emitting diode (LED) or an array of infrared LEDs. Thus, the configuration of the light source **12** shown in FIG. 3 and described above is a preferred embodiment only, and the invention is not limited to any particular configuration of the light source **12.**

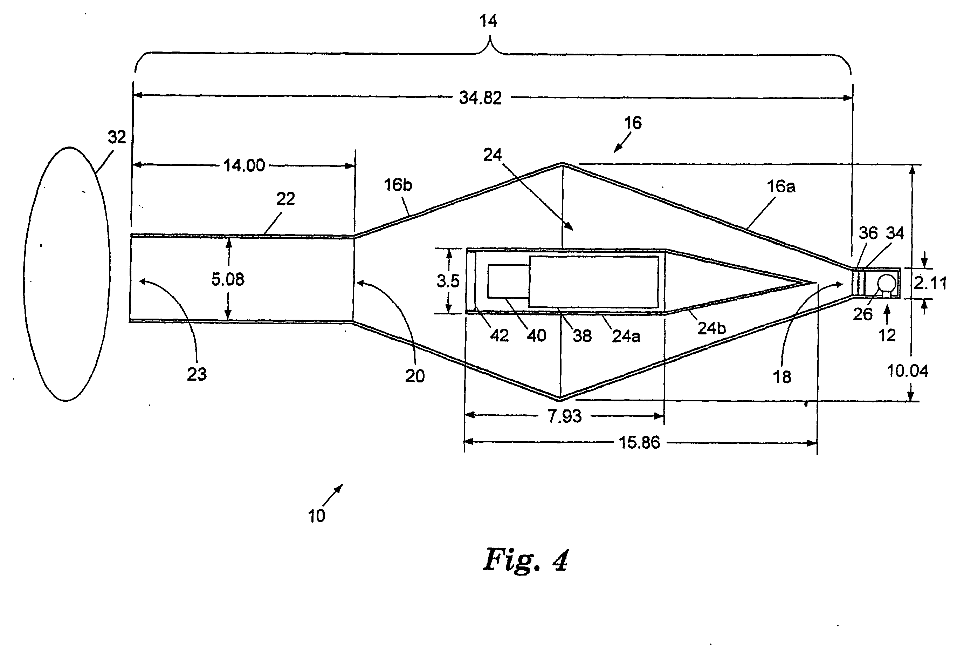

FIG. 4 depicts the dimensions of a preferred embodiment of the illumination system **10.** As shown in FIG. 4, the total length of the light diffusing structure **14** is approximately 34.82 inches. The height and width of the outer enclosure **16** at the juncture of the first and second sections **16a** and **16b** is approximately 10.04 inches. The preferred length of the light guide **22** is approximately 14.00 inches, and its height and width is approximately 5.08 inches. Preferably, the total length of the inner reflector **24** is approximately 15.86 inches. The preferred length of the tubular section **24a** of the inner reflector **24** is approximately 7.93 inches. The height and width of the tubular section **24a** is approximately 3.5 inches. The height and width of the light source **12** is approximately 2.11 inches.

As shown in FIG. 4, a preferred embodiment of the invention includes a lens **40** used in conjunction with the video imaging device **38** to produce a video image of the object **32** based on diffuse light reflected from the object **32.** Preferably, the imaging device **38** of this embodiment is a charge-coupled device (CCD) video camera **38** manufactured by Cohu, having model number 631520010000. The lens **40** of the preferred embodiment is a 25 mm f-0.95 movie camera lens manufactured by Angenieux.

The camera **38** and lens **40** of the preferred embodiment are disposed within the tubular section **24a** of the inner reflector **24.** As shown in FIG. 4, the open end of the tubular section **24a** forms an aperture toward which the camera **38** and lens **40** are pointed. In this manner, the hollow light guide **22** is substantially centered within the field of view of the camera **38.** Thus, the camera **38** receives light reflected from the object **32** that enters the light guide **22,** travels through the enclosure **16,** and enters the open end of the section **24a.**

As shown in FIG. 4, the preferred embodiment of the invention includes an infrared-transmitting filter **42** disposed in the open end of the tubular section **24a.** This filter **42** receives light reflected from the object **32,** and any other light that may enter the enclosure **16,** and substantially eliminates all light having wavelengths outside the infrared range of approximately 700 to 1000 nanometers. In the preferred embodiment, the filter **42** substantially eliminates light having wavelengths outside a selected infrared range of approximately 800 to 850 nanometers. Thus, the light that passes through the filter **42** and into the lens **40** is infrared light within the selected wavelength range. Therefore, the camera **38** primarily receives infrared light which originates from within the illumination system **10** and which is reflected from the object **32.**

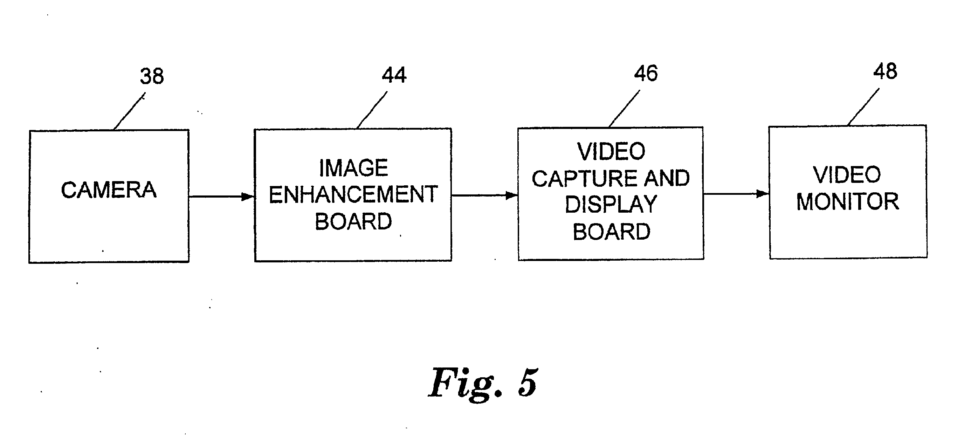

Based on the light reflected from the object **32,** the camera **38** generates a video image of the object **32** in the form of an electrical video signal. As shown in FIG. 5, the video signal is preferably provided to an image enhancement board **44,** such as a board manufactured by DigiVision having a model number ICE-3000. The board **44** generates an enhanced video image signal based on the video signal from the camera **38.** The enhanced video image signal is provided to a video capture and display card **46,** such as a model 20-TD Live card manufactured by Miro. The card **46** captures still images from the image signal which may be saved in digital format on a digital storage device. The card **46** also formats the video image signal for real-time display on a video monitor **48.**

It should be appreciated that the illumination system **10** could use other means for generating diffuse infrared light in accordance with the invention. For example, the light providers **10a-10f** of FIG. 1 could be embodied by a ring-light strobe light. Alternatively, a circular array of LEDs could be used to illuminate a plastic transmitting diffuser placed near the surface of the object **32.** In the latter embodiment, the light providers **10a-10f** would correspond to the individual LEDs in the array.

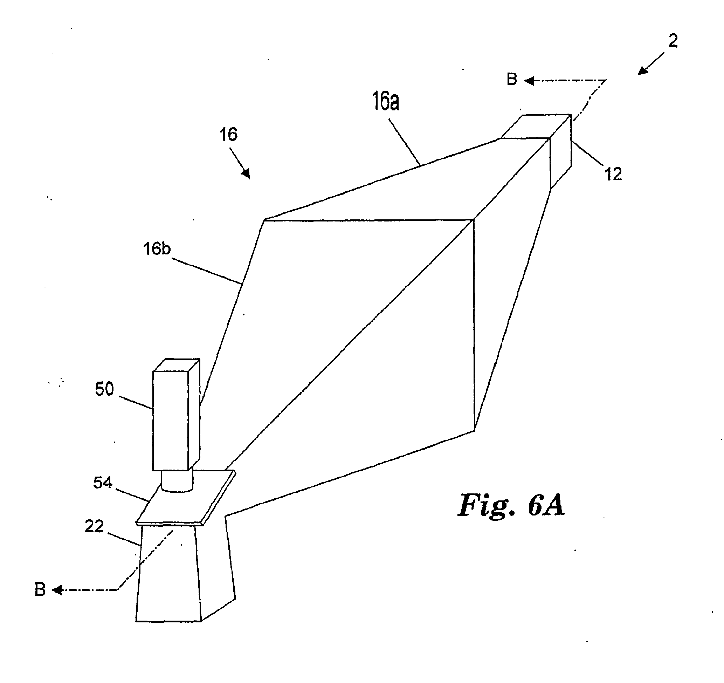

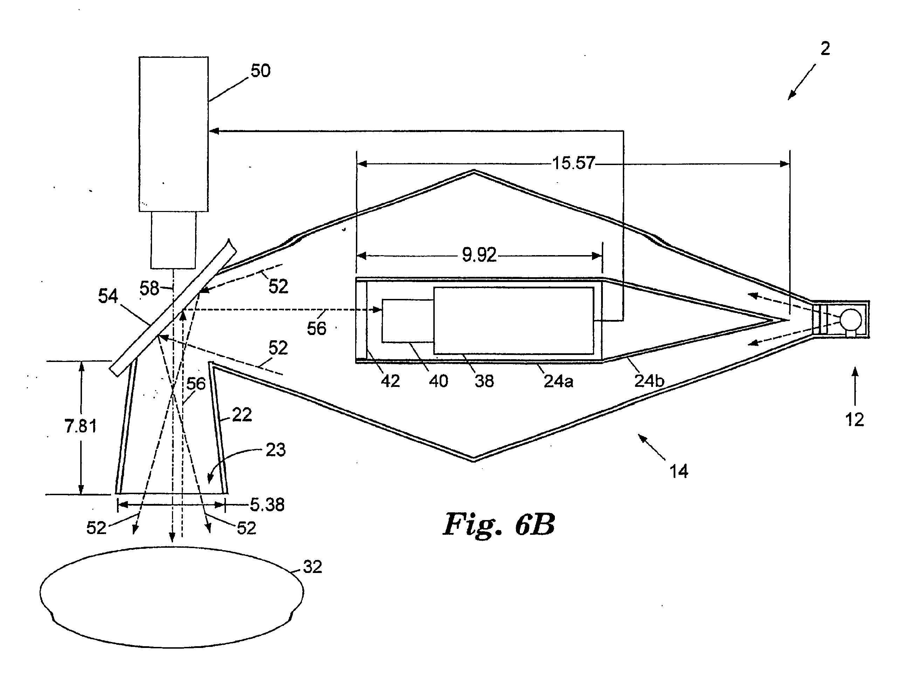

In an alternative embodiment of the invention depicted in FIGS. 6a and 6b, the imaging system **2** includes a video projector **50** for illuminating the object **32** with an image of the object **32** to enhance the visual contrast between lighter and darker areas of the object **32.** As described in U.S. Pat. No. 5,969,754, entitled CONTRAST ENHANCING ILLUMINATOR, the features of an object are visually enhanced for an observer when the features of a projected visible-light image of the object overlay the corresponding features of the object. The overlaid visible-light image causes the bright features of the object to appear brighter while the dark areas remain the same.

The embodiment of the invention shown in FIGS. 6a and 6b provides diffuse infrared light (represented by the rays **52**) to the object **32** in a manner similar to that described previously. However, in the embodiment shown in FIGS. 6a and 6b, the optical path of the illuminating light is folded, such that the exit aperture **23** of the light guide **22** is rotated by 90 degrees relative to the exit aperture shown in FIGS. 1-3.

As shown in FIG. 6b, a beam separator, such as a hot mirror **54,** receives infrared light **52** from the interior of the light diffusing structure **14** and reflects the infrared light **52** into the light guide **22** and toward the object **32.** The hot mirror **54** also receives an infrared image of the object **32** (represented by the ray **56**) and reflects it toward the camera **38.** The hot mirror **54** receives the visible-light image (represented by the ray **58**) from the projector **50** and transmits it into the light guide **22** and toward the object **32.**

As explained in greater detail in U.S. Pat. No. 5,969,754, the video output signal from the video camera **38** is provided as a video input signal to the projector **50.** Based on the video input signal, the projector **50** projects the visible-light image **58** of the object **32** toward the hot mirror **54.** The hot mirror **54** receives the visible-light image **58** and transmits it into the light guide **22** toward the object **32.** By proper alignment of the projected visible-light image **58** from the projector **50** with the infrared image **56** of the object **32** which is sensed by the camera **38,** the features in the projected visible-light image **58** are made to overlay the corresponding features of the object **32.**

When the object **32** is body tissue, and the invention is used to find subcutaneous blood vessels in the body tissue, the blood vessels appear as dark lines in the projected visible-light image **58.** Thus, when the visible-light image **58** is projected onto the body tissue, the subcutaneous blood vessels will lie directly beneath the dark lines in the projected visible-light image **58.** In this manner, the invention significantly improves a medical practitioner's ability to find subcutaneous blood vessels while minimizing discomfort for the patient.

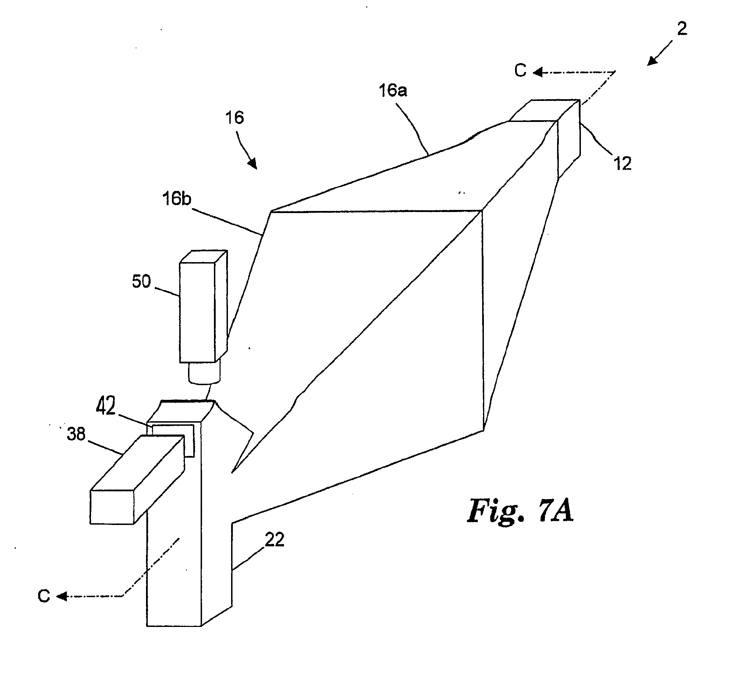

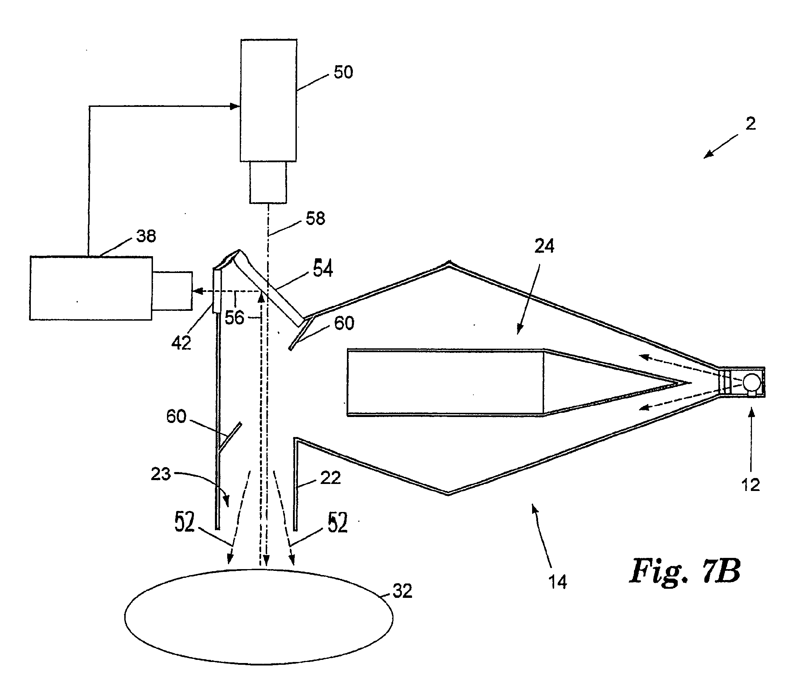

FIGS. 7a and 7b depict an alternative embodiment of the invention for use as a contrast enhancing illuminator. The embodiment of FIGS. 7a-b operates in a fashion similar to the embodiment of FIGS. 6a and 6b. However, in the embodiment of FIGS. 7a-b, the camera **38** is located outside the light diffusing structure **14.** To accommodate the different location of the camera **38,** the hot mirror **54** shown in FIGS. 7a-b is rotated by 90 degrees clockwise relative to its position in FIGS. 6a-b. Otherwise, the hot mirror **54** serves a similar function as that described above in reference to FIGS. 6a-b. Also to accommodate the different camera location, the infrared-transmitting filter **42** is mounted in a wall of the light guide **22.** A reflective panel **60** is provided in this embodiment to further direct the light from the light source **12** into the light guide **22** and toward the exit aperture **23.** Preferably, the panel **60** is a flat reflective sheet having an orifice therein to allow light to pass between the object **32** and the camera **38** and projector **50.**

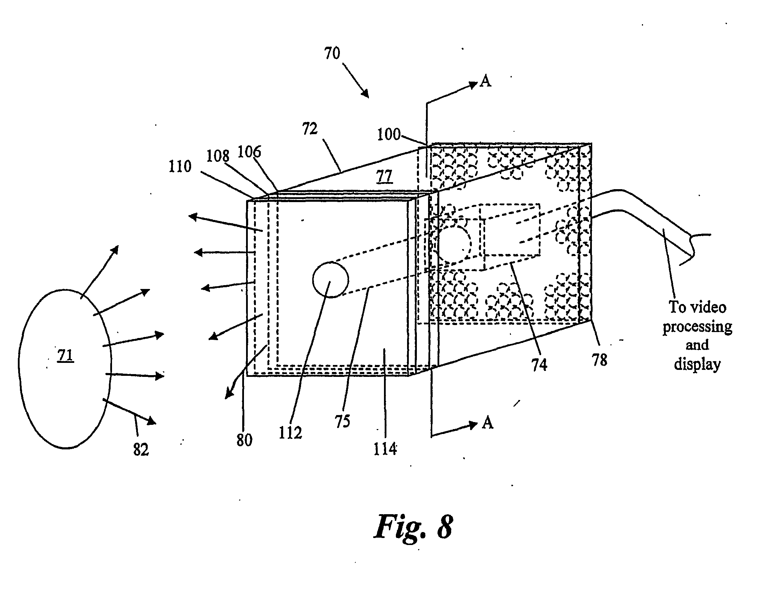

A preferred embodiment of a relatively compact and highly reliable imaging system **70** is depicted in FIGS. 8-11. The imaging system **70** is most preferably configured to illuminate an object **71,** such as body tissue and the like, and to produce a video image of the object **71** based upon infrared light reflected from the object **71.** The imaging system **70** preferably includes a housing **72** which contains the imaging features of the system **70.**

As shown in FIG. 8, the housing **72** preferably has a substantially rectangular configuration. The housing **72** preferably has a length of between about three and about five inches and a width of about three and one-half inches. It will be appreciated by those skilled in the art that the imaging system **70** can be configured in a variety of ways and the invention should not be limited by any specific examples or embodiments discussed herein. For example, in FIG. 8 the housing is depicted as being substantially rectangular, however, circular, polygonal, and other geometries and sizes are feasible as well.

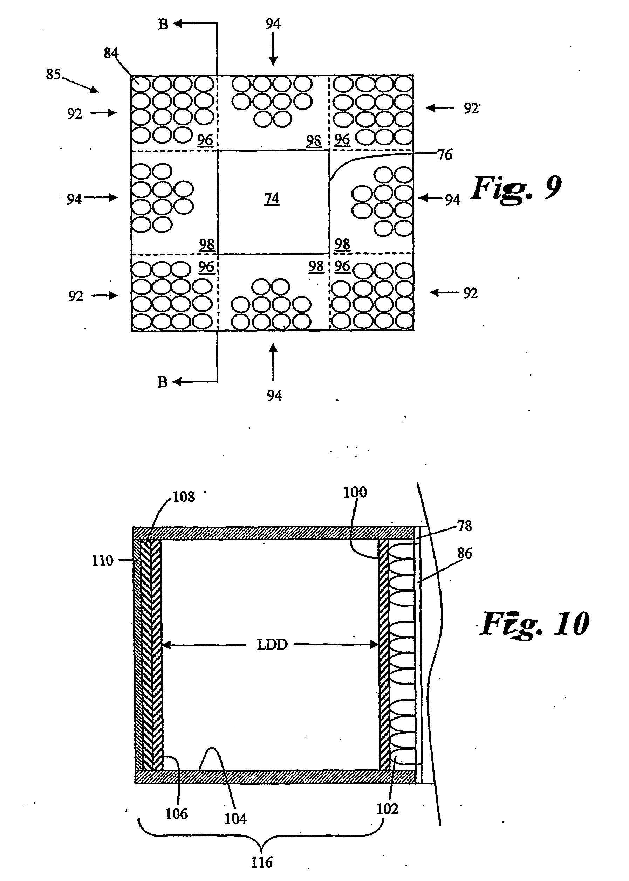

An imaging device **74,** such as a video camera having a lens **75,** and video processing components reside within the housing **72.** The imaging device **74** and video processing components operate to detect infrared light and to process the detected infrared light from the object **71.** The imaging system **74** produces an image based on the detected infrared light reflected from the object **71,** as described herein. As shown in FIGS. 8 and 9, the imaging device **74** is preferably mounted within an aperture **76** of mounting wall **78,** with the lens **75** extending into the housing interior **77,** as described further below. More particularly, the camera **74** is preferably centrally and symmetrically mounted within the housing **72.** This preferred symmetrical camera location tends to maximize the amount of light detected by the camera, which enhances the image produced by the system **70,** thereby enhancing the illumination of blood vessels disposed below subcutaneous fat in body tissue.

The housing **72** most preferably contains various components operable to transmit diffuse light from the system **70** toward the object **71.** Arrows **80** represent diffuse light transmitted by the system **70.** Arrows **82** represent the light reflected from the object **71.** As shown in FIG. 9, as viewed in the direction of the arrows along the section line A-A of FIG. 8, the wall **78** contains a number of infrared light emitting diodes (LEDs) **84** disposed in a LED array **85** for emitting infrared light. The LED array **85** defines a LED plane of reference. When activated, each LED **84** preferably transmits light at a wavelength of about 740 nanometers (nm). In the preferred embodiment, each LED **84** is manufactured by Roithner Lasertechnik of Austria under model number ELD-740-524.

As shown in FIG. 10, and according to the preferred embodiment, the LEDs **84** are mounted on a circuit board **86** located adjacent to wall **78.** As shown in FIG. 9, there are most preferably eight groups **92, 94** of LEDs **84** concentrically arranged about the imaging system **74.** The concentric LED arrangement tends to provide maximal dispersion and transmission of diffuse light from the system **70.** It is preferred that each group **92, 94** of LEDs **84** contain at least ten LEDs **84.** However, the system **70** can include more or fewer LEDs within a particular group depending upon a desired implementation of the system **70.** Furthermore, the system **70** can include more or fewer groups of LEDs in the LED array **85.**

With continuing reference to FIG. 9, there are four groups **92** of LEDs **84** located about the corner regions **96** of the LED array **85.** Most preferably, at least fifteen LEDs **84** are disposed in each corner region **96** of the LED array **85.** There are preferably four groups **94** of LEDs **84** disposed in lateral regions **98** of the LED array **85.** Each lateral region **98** is located substantially between each corner region **94.** Most preferably, at least ten LEDs **84** are disposed in each lateral region **98** of the LED array **85.**

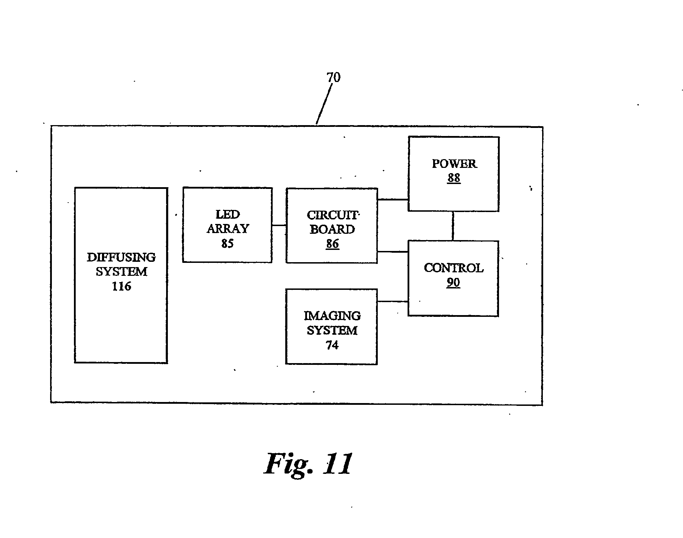

As described above, the LED array **85** is most preferably disposed on circuit board **86.** In conjunction with the control system **90,** the circuit board **86** includes control circuitry that controls the activation of one or more LEDs **84** within a particular group or groups **92, 94** of LEDs **84** in the LED array **85.** As shown in the block diagram of FIG. 11, a power source **88** and a control system **90,** such as a microprocessor or similar control device, are electrically connected to the circuit board **86.** It will be appreciated that is also possible to control the LEDs without using a control system **90,** that is, power source **88** can be switched "on" or "off" to activate and deactivate the LED array **85.** It will be appreciated that pulse modulation techniques can also be used in conjunction with power source **88** to activate and deactivate one or more of the LEDs **84** in the LED array **85** according to a preferred duty cycle, herein defined as the LED "on" time relative to the LED "off" time.

As shown in the block diagram of FIG. 11, in a preferred embodiment of the imaging system **70,** the LED array **85** is electrically connected via circuit board **86** to the power source **88** and control system **90.** The control system **90** includes control features for controlling the LED array **85** to emit infrared light toward an object **71.** As described herein, the control system **90** can enable one or more of the LEDs **84** in a group or groups of the LED array **85** to emit light continuously or intermittently. That is, one LED **84** or a plurality of LEDs **84** can be selected and controlled to emit infrared light intermittently or continuously toward the object **71.** Thus, the system **70** can be configured to transmit infrared light from the LED array in various permutations and combinations of LEDs **84** and/or LED groups **92, 94.**

Referring now to FIG. 10, a first diffusion layer **100** is disposed adjacent to the emitting surfaces **102** of the LEDs **84** in the LED array **85.** According to a preferred embodiment, the first diffusion layer **100** is glued, such as using known adhesives, onto the emitting surfaces **102** of the LED array **85,** thereby operating to diffuse the light emitted by one or more LEDs **84** in the LED array **85.** The first diffusion layer **100** is most preferably a holographic twenty degree diffuser, such as a product having identification code LSD20PC10-F10x10/PSA, manufactured by Physical Optics Corporation of Torrance, Calif. Most preferably, the first diffusion layer **100** has a length of about three and one-half inches, a width of about three and one-half inches, and a thickness of about 0.10 inches. When one or more of the LEDs **84** in the LED array **85** are activated, the first diffusion layer **100** diffuses the infrared light emitted from the LED array **85,** thereby providing a first amount of diffusion to the emitted infrared light.

The interior surfaces **104** of the housing **72** are shown in FIG. 10. Most preferably, the interior surfaces **104** are coated with a reflective coating, such as white paint or the like, which reflects and further diffuses the already diffuse light produced by the first diffusion layer **100.** With continuing reference to FIG. 10, a second diffusion layer **106** is spaced apart from the first diffusion layer **100** by a distance LDD. Most preferably, the distance LDD between the first and second diffusion layers **100** and **106** is about three inches. The second diffusion layer **106** is most preferably a holographic twenty degree diffuser, similar to or the same as the above-described first diffusion layer **100.** The second diffusion layer **106** has a preferred length of about three and one-half inches, a width of about three and one-half inches, and a thickness of about 0.10 inches.

The second diffusion layer **106** further diffuses the already diffuse light reflected from the interior surfaces **104** and provided by the first diffusion layer **100.** As shown in FIG. 8, the first and second diffusion layers are substantially planar, that is, the layers **100** and **106** each define a planar geometry. According to the most preferred embodiment, the planes defined by the first and second diffusion layers **100** and **106** are substantially parallel with respect to one another. The preferred parallel planar arrangement of the diffusion layers **100, 106** tends to promote a quantifiable and uniform amount of diffuse light emanating from the system **70** when one or more of the LEDs **84** are enabled.

With continuing reference to FIG. 10, a backing material **108,** such as LUCITE material sold under the trademark LUCITE and manufactured by DuPont of Wilmington, Delaware, is disposed adjacent to the second diffusion layer **106.** Most preferably, the backing material has a thickness of about 0.125 inches. A visible polarizer **110** is disposed adjacent to the backing material **108.** The visible polarizer **110** is most preferably manufactured by Visual Pursuits of Vernon Hills, Illinois, under part number VP-GS-12U, and having a thickness of about 0.075 inches.

Thus, the system **70** is operable to produce various levels of diffusion as the emitted light progresses through the first diffusion layer **100,** reflects off of the interior surfaces **104** of the first compartment **72a,** and continues to progress through the second diffusion layer **106,** backing material **108,** and polarizer **110.** Thus, a level of diffusion results after the emitted light passes through the first diffusion layer **100.** Another level of diffusion results from the reflection from the interior surfaces **104** of the first compartment **72a** of the already diffused light provided by the first diffusion layer **100.** Yet another level of diffusion results after the diffuse light passes through the second diffusion layer **106.**

As shown in FIG. 8, the visible polarizer **110** preferably includes a central portion **112,** most preferably in the shape of a circle having about a one-inch diameter. The central portion **112** geometry most preferably coincides with the shape and dimension of the camera lens **75.** The polarization of the central portion **112** is preferably rotated approximately ninety degrees with respect to the polarization of the surrounding area **114** of the polarizer **110.** In the preferred embodiment, the camera lens **75** contracts the backing material **108.** As shown in FIG. 8, the positional location of the lens **75** within the housing **70** preferably coincides with or shares the same central axis as the central portion **112** of the polarizer **110.** The central portion **112** of the polarizer **110** coinciding with the front of the lens **75** tends to remove any surface glare ("specular reflection") in the resulting camera image.

As shown in FIG. **10****,** the backing material **108** and the visible polarizer **110** have planar surfaces which preferably include a similar planar orientation with respect to the planes defined by the first and second diffusion layers **100, 106.** According to a most preferred embodiment, the first diffusion layer **100,** interior surfaces **104,** second diffusion layer **106,** backing material **108,** and visible polarizer **110** define a diffusing system **116** (FIG. 10) for providing diffuse light to an object **71.** It will be appreciated that the diffusing structure can include more or fewer components and the invention is not to be limited by any specific examples or embodiments disclosed herein. For example, the diffusing system **116** can include either the first or the second diffusion layers **100, 106,** with or without the polarizer **110,** or can include the first and second diffusion layers **100, 106** without the polarizer **110.**

Once actuated, the system **70** operates to transmit diffuse light **80** toward an object **71** and produce a video image of the object **71** with the imaging system **74,** as described above. More particularly, once the power source **88** is enabled, one or more of the LEDs **84** in the LED array **85** emit infrared light from the emitting surface(s) **102.** The first diffusion layer **100** provides a first amount of diffusion to the emitted infrared light. The interior surfaces **104** further diffuse the diffuse light emanating from the first diffusion layer **100.** The second diffusion layer **106** further diffuses the already diffuse light which is then transmitted through the backing material **108** and the polarizer before illuminating the object **71.** As described above, the object **71** reflects the emitted diffuse light **80** producing diffuse reflected light **82** that is captured by the imaging system **74.** The imaging system **74** then produces a video image of the object **71.** Accordingly, by emitting diffuse light according to a unique diffusion providing system **70,** the system **70** aids in locating and differentiating between different material properties of the object **71,** such as between blood vessels and tissue.

It is contemplated, and will be apparent to those skilled in the art from the preceding description and the accompanying drawings, that modifications and/or changes may be made in the embodiments of the invention. For example, the planes defined by the first or second diffusing layers **100** and **106** can be adjusted to not be parallel with respect to one another, thereby providing different levels of diffuse light from the system **70.** Furthermore, the plane defined by the LED array **85** is most preferably in substantial parallel relation with respect to the plane defined by the first diffusing layer **100.** However, the planes defined by LED array **85** and the first diffusing layer **100** can be varied to accommodate various operational conditions, as will be appreciated by those skilled in the art. Accordingly, it is expressly intended that the foregoing description and the accompanying drawings are illustrative of preferred embodiments only, not limiting thereto, and that the true spirit and scope of the present invention be determined by reference to the appended claims.

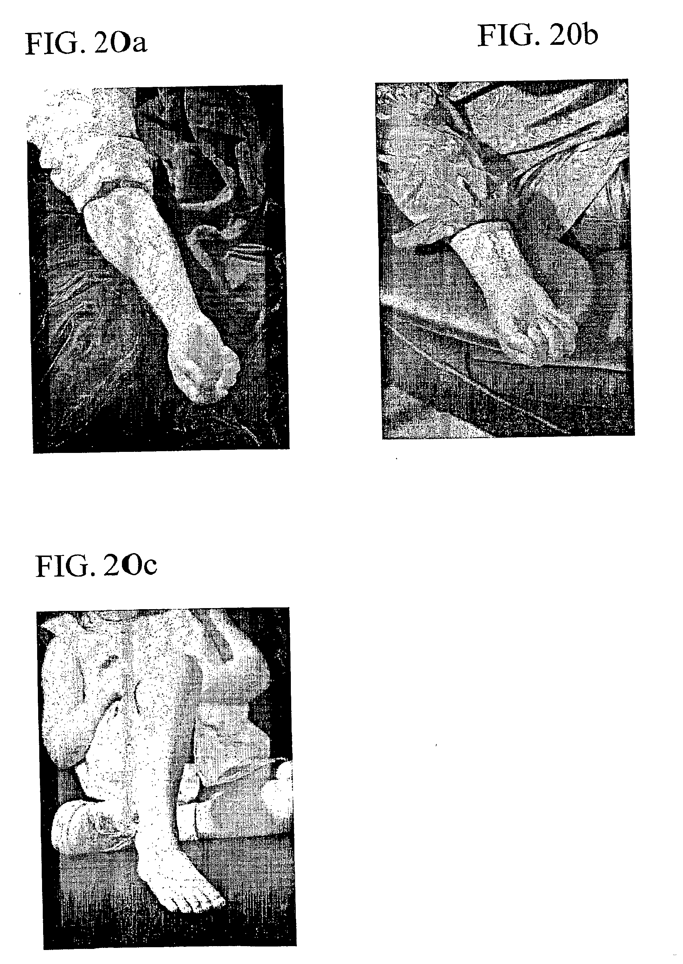

FIGS. 20a, 20b, and 20c are photographs of test subjects showing processed images of subcutaneous blood vessels being projected onto the surface of each subject's body tissue which covers the viewed blood vessels.

Additional embodiments will now be described showing a variety of configurations of illumination sources, imaging devices for viewing the image of buried structure beneath the surface of the illuminated obj ect, and projectors for projecting the processed image back onto the surface of the object. Because all of the embodiments of the present invention have many structural features in common, only the differences between the structures need be discussed in detail, it being understood that similar structural features of all the embodiments perform similar functions. Those skilled in the art will readily recognize the similar structural features that appear in all embodiments of the present invention.

Because of the present invention's departure from the prior art by projecting the image of the buried structure back onto the surface of the object (rather than onto a screen or monitor that is remote from the surface of the object), an observer using the present invention is not subject to the parallax errors that otherwise occur with prior art devices if an observer were to view from off-axis. An important feature of all embodiments is that the image of buried structure viewed by the imaging device should be substantially within a first spectrum outside a second spectrum of the image that is projected back onto the surface of the object, thereby causing the imaging device to be blind to the image that is projected back onto the surface of the object. The substantial non-overlap of the spectrum of the viewed image of the buried structure with the spectrum of the projected image of the buried structure effectively decouples the image processing of the buried structure's image from interference by the projected image. Because the projected image is in the visible light spectrum and the illumination of the object for the imaging device is in the infrared spectrum, a substantial non-overlap of the two spectrums is maintained. In another herein-disclosed embodiment, rather than illuminating the object with light that is primarily in the infrared spectrum, the object can be illuminated by broad-spectrum ambient light, and an infrared filter is placed in front of the imaging device to remove all spectral components outside the infrared spectrum, thereby causing the imaging device to only see the infrared component of the broad-spectrum diffuse light reflected from the object.

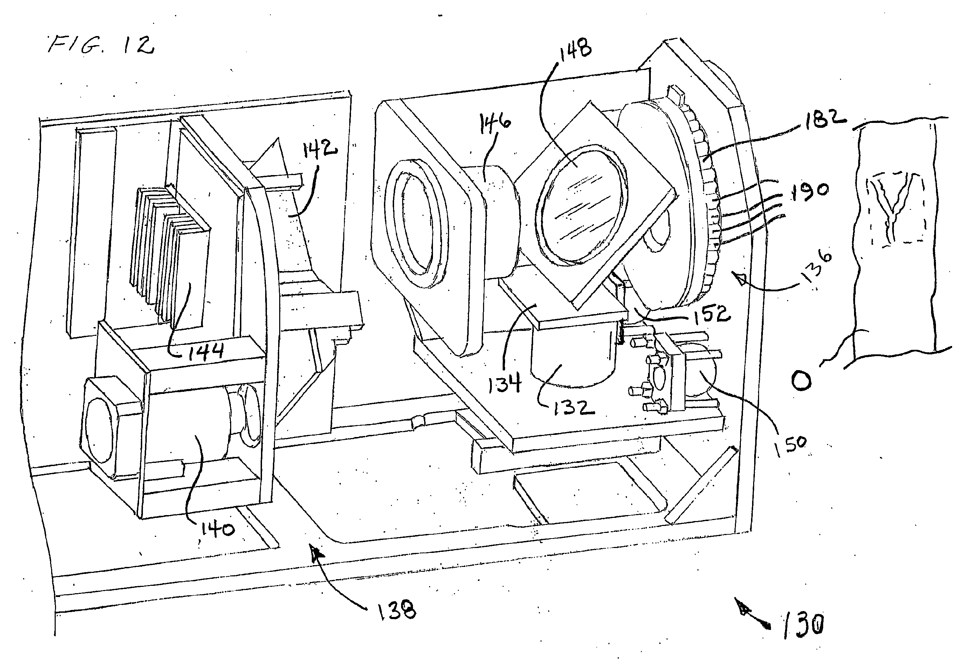

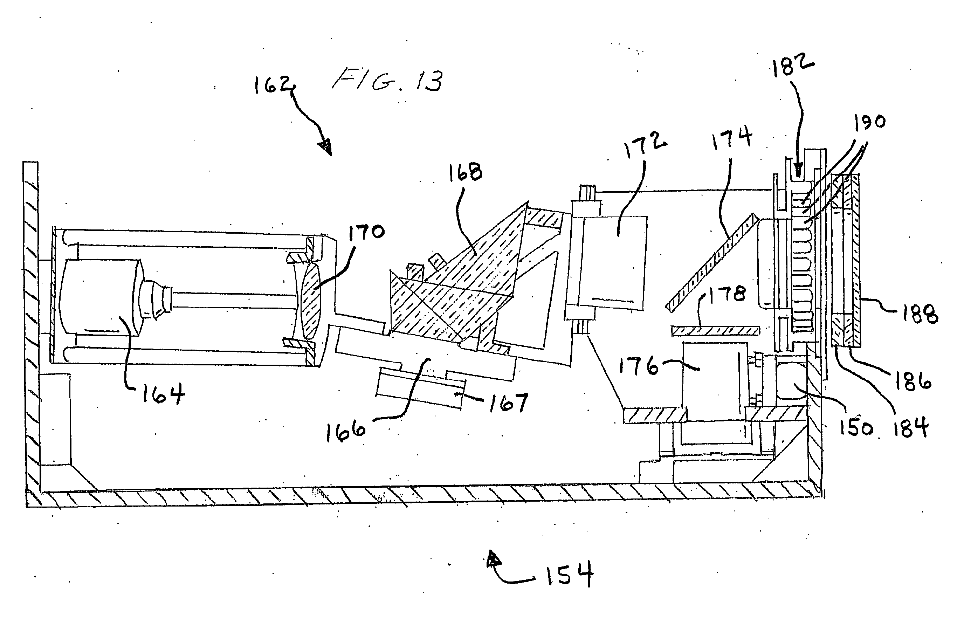

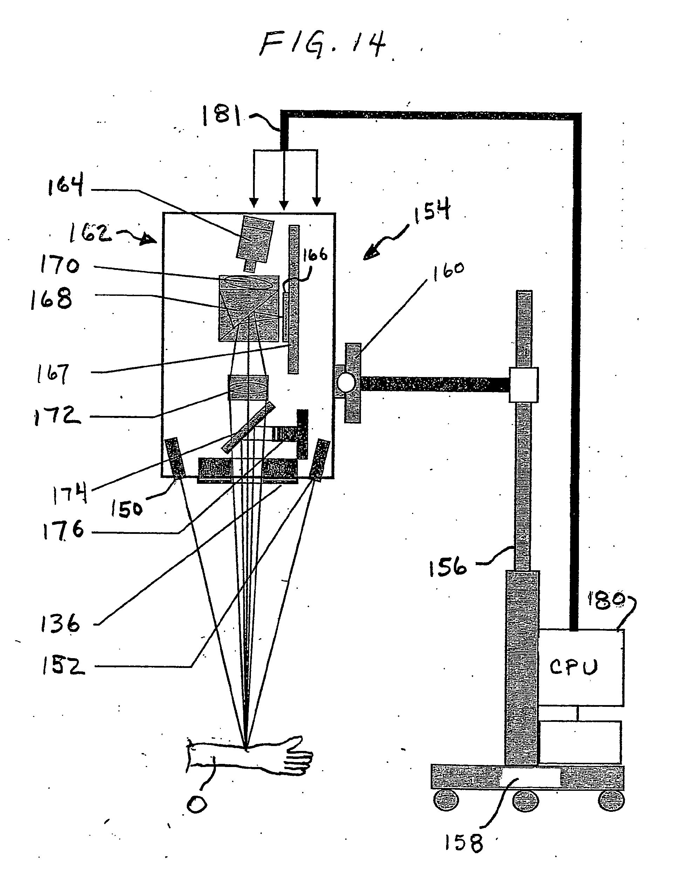

A third preferred embodiment **130** of the imaging system is shown in FIG. 12. The near-infrared illumination A well-known CCD camera with lens **132** is used as the imaging device, as in all embodiments. A second polarizing filter **134** is interposed between the CCD camera and the reflected light from the viewed object, as previously described for earlier embodiments, so as to reduce specular reflection from the surface of the object. The illumination source, first polarizing filter, holographic illumination diffuser ring, and optically-neutral glass cover, all generally at **136,** are best described below in the discussion of the fourth embodiment of the imaging system shown in FIGS. 13 and 14, which has the same structure **136** which is shown in cross-section for that embodiment.

As with all embodiments, the third preferred embodiment includes a well-known video projector **138** or so-called "light engine" for projecting a visible image onto the object **O** under examination. A desirable feature of the video projector **138** is high output light intensity, because the intensity of the output of the projector's light is a determining factor in how well the projected image can be viewed under normal room illumination. Video projector **138** includes a high-intensity green LED light source **140** which emits light into well-known prism assembly **142,** thereby causing the emitted light to fold back, by internal reflection within prism assembly **142,** and be directed rearwardly toward well-know Digital Light Processing ("DLP") device **144,** also known as a Digital Minor Device ("DMD"), having an array of closely-packed small mirrors that can individually shift the direction of the light beam reflected therefrom so as to either cause the light beam to be directed toward the target object through well-known projection lens **146** or to cause the light beam to not be directed toward the target object, thereby turning the emitted light beam off on a pixel-by-pixel basis in a manner well-known to those skilled in the art. It shall be understood that prism assembly **142** permits a more compact apparatus for the various embodiments of the imaging system, and the use of such prism assemblies is well known to those skilled in the art of video projectors.

As with the prior-described embodiments, a well-known so-called "hot mirror" **148** is interposed at 45 degrees to intercept the infrared light reflected from the viewed object and reflect that infrared light downward to camera **132.** "Hot mirror" **148** acts as a mirror to longer wavelengths of light (such as infrared light) but higher-frequency light, such as the green light from projector **138,** passes through without reflection and toward the viewed object.

Imaging system **130** further has first and second lasers **150, 152** for ensuring that the target is properly located for in-focus viewing by camera **132,** as hereinafter described.

Referring now to FIGS. 13 and 14, a fourth embodiment **154** of the imaging system of the present invention will now be explained.

Fourth embodiment **154** is mounted upon a pole **156** that extends upwardly from a mobile cart **158,** allowing the imaging system **154** to be easily transported. A fine-focus stage **160** allows imaging system **154** to be raised or lowered so that it is properly positioned above the target object **O.** As with all embodiments, video projector **162** is provided with a 525 nm green LED illumination source ("photom engine") **164** for illuminating the DMD / DLP chip **166.** A suitable photon engine **164** for use with the fourth embodiment is the Teledyne Lighting model PE09-G illuminator, having an output intensity of 85 lumens. DMD chip **166** may be a Texas Instruments part number 0.7SVGA SDR DMD chip having a resolution of 848 x 600 pixels and a mirror tilt angle of ten degrees and a frame rate of 30 Hz. Well-known prism assembly **168,** as before, internally reflects the light from photon engine **164** toward DMD chip **166** and then directs the light reflected from DMD chip **166** toward object **O.** DMD chip **166** is controlled by a well-known drive electronics board **167** which may be made by Optical Sciences Corporation.

Interposed between photon engine **164** and prism assembly **168** is a condenser lens **170** such as a BK7 Bioconvex lens, part number 013-2790-A55, sold by OptoSigma, having a BBAR/AR coated surface coating for 425-675 nm light. As the projector light emerges from prism assembly **168,** it passes through well-known projection lens **172,** Besler part number 8680 medium format enlarger lens and then through well-known "hot-mirror" (high pass filter) **174,** which reflects the received infrared light image from the object **O** through second polarizing filter **178** ant then to camera **176.** A suitable camera **176** is the Firefly Camera, part number FIRE-BW-XX, sold by P oint Grey Research, which uses a 640 x 480 CCD chip, part number Sony ICX084AL, and which communicates its images to computer ("CPU") **180** through an IEEE-1394 ("FireWire") interface. It should be noted that computer **180** has a number of interfaces signals **181** that communicate with the imaging system in a manner well-known to those skilled in the art. As briefly mentioned for the third embodiment, the fourth embodiment also has first and second lasers **150, 152** for ensuring that the target **O** is properly located for in-focus viewing by camera **176.**

As with third embodiment **130** shown in FIG. 12, and with reference to FIGS. 12, 13, and 14, fourth embodiment **154** has an assembly **136** that includes infrared illumination source **182,** first polarizing filter **184** (which is ring-shaped with a center hole therethrough so as not to affect the projected image from projector **162** or the viewed image of the object), holographic illumination diffuser ring **186** (which likewise has a center hole therethrough for passage of the projected image from projector **162** and of the viewed image of the object) and which diffuses the light from LEDs **190,** and optically-neutral glass cover **188.** Infrared illumination source **182** is a group of LEDs preferably arranged in a select pattern, such as a circular ring having a centrally-disposed hole through which the projected image and the viewed object's image passes. The LEDs are preferably 740 nm near-infrared LEDs **190** that illuminate the object **O,** and research has determined that such a structure provides sufficient diffused infrared light for satisfactory illumination of object **O.**

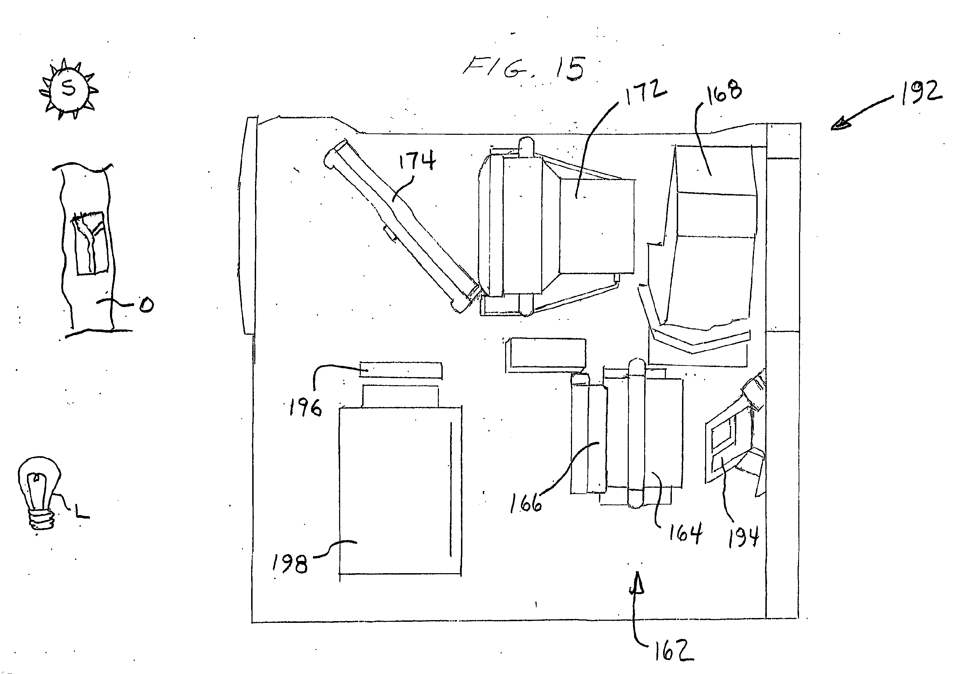

Referring to FIG. 15, a fifth embodiment **192** of the imaging system of the present invention will now be explained. The significant difference between this fifth embodiment and the other embodiments is that the fifth embodiment does not provide an integral diffuse infrared light source (*e*.*g*., illumination source **182** with a ring of LEDs **190**) for illuminating the object, but instead views the object as illuminated by ambient light **L** (or the sun **S**) that has a broader spectrum than the integral diffuse infrared illumination sources heretofore disclosed. While ambient light has some infrared spectral components and is quite diffuse, those infrared spectral components are generally of lower intensity than the infrared light produced by the diffuse infrared illumination sources heretofore disclo sed. Accordingly, a better (*i*.*e*., more sensitive) image device camera is required for this embodiment, with better optics than the previously-described embodiments.

Like the other embodiments, the fifth embodiment **192** includes video projector **162,** including a green "photon engine" **164,** prism assembly **168,** projector lens **172,** and DMD chip **166.** To permit a compact design, fifth embodiment **192,** as could any of the embodiments, includes a "fold mirror" **194** that folds the beam at a right angle within the projector between the photon engine **164** and prism assembly **168.** Also like the other embodiments, fifth embodiment **192** includes a "hot mirror" **174.**

Fifth embodiment **192** further has an infrared filter **196** interposed in the optical path between the imaging device (camera **198**) and object **O** so as to filter out all but the infrared component of the image viewed by camera **198.** Camera **198** is preferably a Basler CMOS camera, model A600-HDR, made by Basler Vision Technologies of Germany, which has an IEEE 1394 ("FireWire") interface and allows capture of images with up to a 112 dB dynamic range. An advantage of the fifth embodiment is that it can be (and should be) used in a brightly-illuminated room.

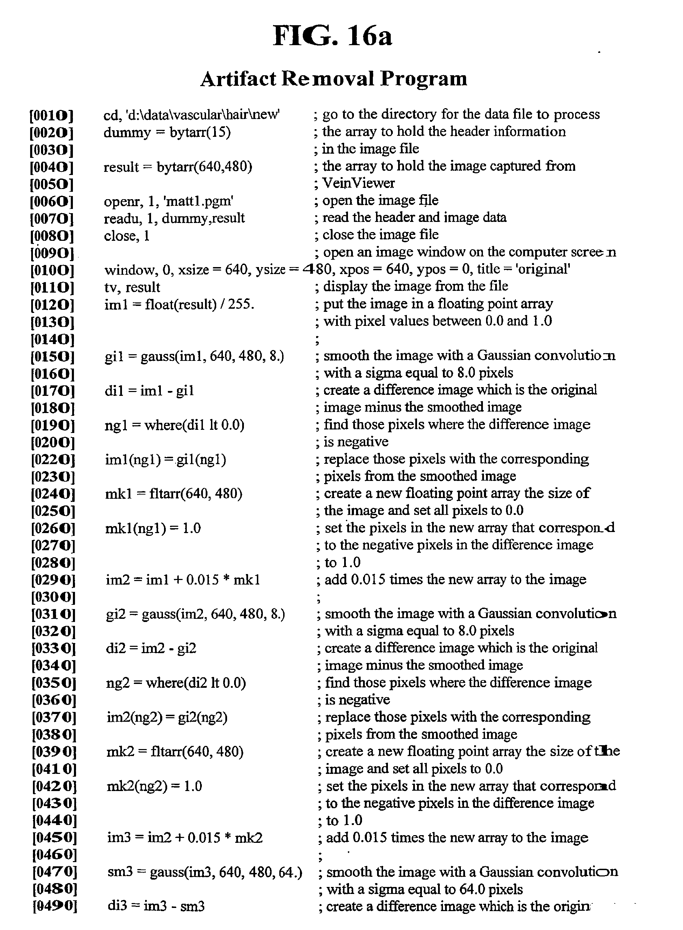

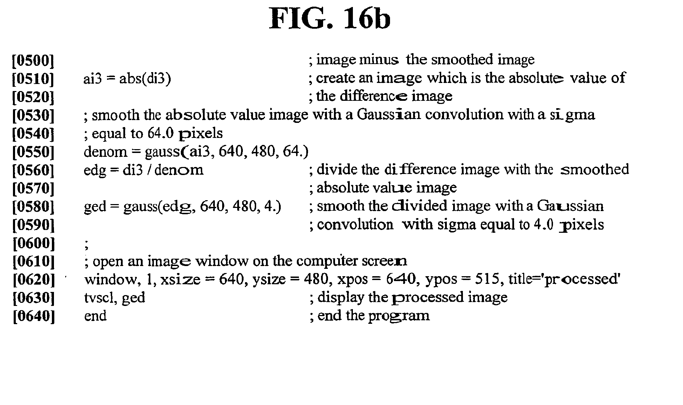

Experimental testing has revealed that some persons have arms or legs that are so covered with surface hair that it is difficult to see with clarity the projected subcutaneous structure that is projected onto the surface of the skin. Investigation has revealed that all hairs, even white hairs, look black in the near infrared. Hence, image processing is performed on the received image in order to remove small dark artifacts, such as hairs, from the image while retaining larger dark objects to maintain the visibility of the veins. FIGS. 16a and 16b, taken together in sequence, are a program listing for artifact removal image processing of the received image. The same artifact removal procedure is performed twice, and then a well-known adaptive edge enhancement procedure is performed, such as, for example, unsharp masking, followed by a smoothing to clean up image artifacts produced by the hair removal. The program listing is well-commented and explains to those skilled in the art the image processing steps that are applied to the image.

The received image, having integer pixel values in the range (0 ... 255) is converted to floating point values between 0.0 and 1.0, inclusive. The resulting image is then converted to smoothed (blurred) using a Gaussian convolution having a sigma of 8 pixels. This is a fairly small value of sigma, and leave small features, such as narrow hairs, in the resulting smoothed image. A "difference image" is created which is the original image minus the Gaussian-smoothed image, producing a zero-centered set of values from - 1.0 to 1.0. Hairs, even white hairs, appear black in the near infrared, so negative pixel values are indicative of hairs, and those negative-value pixels are thus replaced with the corresponding pixels from the Gaussian-smoothed image. This is the first step in the processing of the received image. Next, an array of values is created for the image, such that all pixel locations where the original "difference image" was negative (the "hair" locations) are set to 1.0, and all other pixel locations are set to zero, thereby creating an array populated by 0.0 or 1.0 values, with every "hair pixel" having a value of 1.0 and all others having a zero value. The original image ("im1"), having pixel values ranging from 0.0 to 1.0, is then "boosted" at every "hair pixel" location by 0.015. Because this is a highly non-linear operation, the amount of "boost" if quite small, just 1.5%.

This same set of operations (Gaussian smoothing with a sigma of 8 pixels, creation of a difference image, identifying negative pixel locations, and "boosting" the image where negative pixels (small features and noise) are found) are performed again, and the resulting image is then smoothed again with a Gaussian convolution having a sigma of 64 pixels. A third difference image is created, which is the again-"boosted" image minus the smoothed image, and an image is created that is formed from the absolute value of every pixel in the third difference image. The resulting absolute value image is then smoothed with a Gaussian convolution having a sigma of 64 pixels, and the third difference image is then divided by the smoothed absolute value image, and the resulting divided image is smoothed with a Gaussian convolution having a sigma of 4 pixels.

The foregoing Artifact Removal algorithm allows the contrast to be set by the contrast of the subcutaneous vein (the subsurface structure of interest), ignoring the artifacts (hairs), and thereby prepares the image for adaptive unsharp masking edge enhancement to set the contrast of the final image. Parameters such as sigma values, thresholds, etc., may be varied depending on the age of the subject, degree of pigmentation, etc.

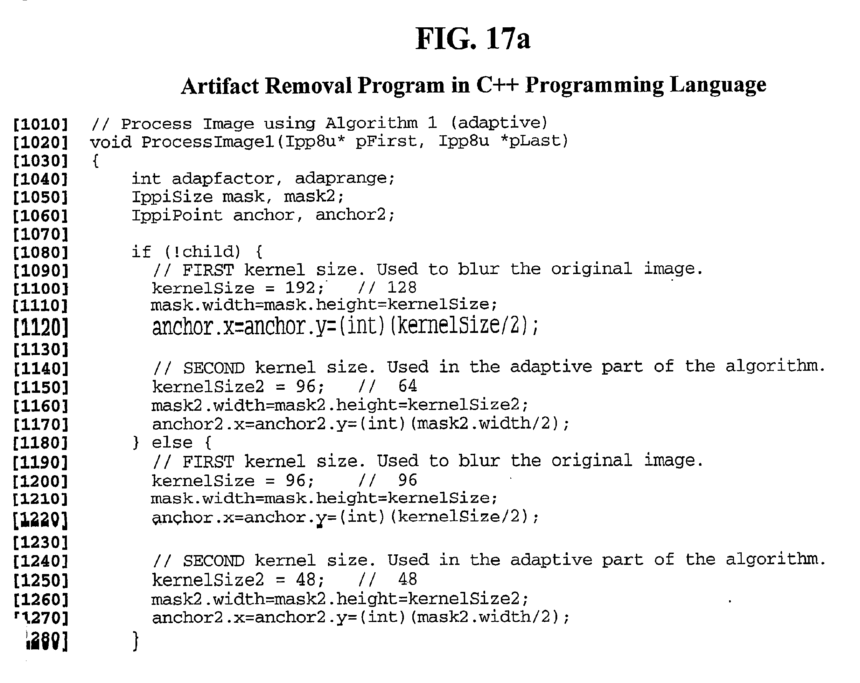

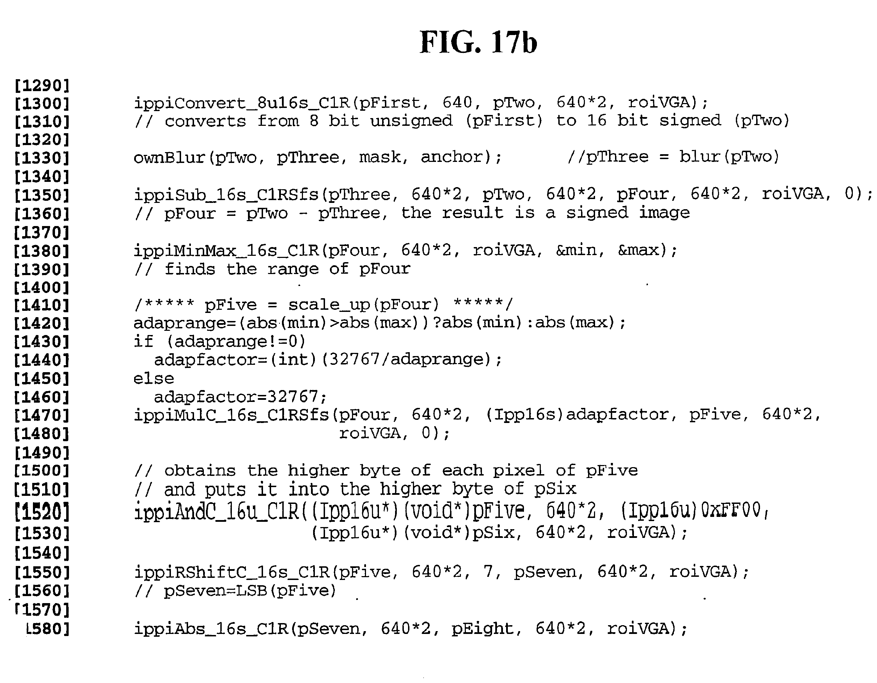

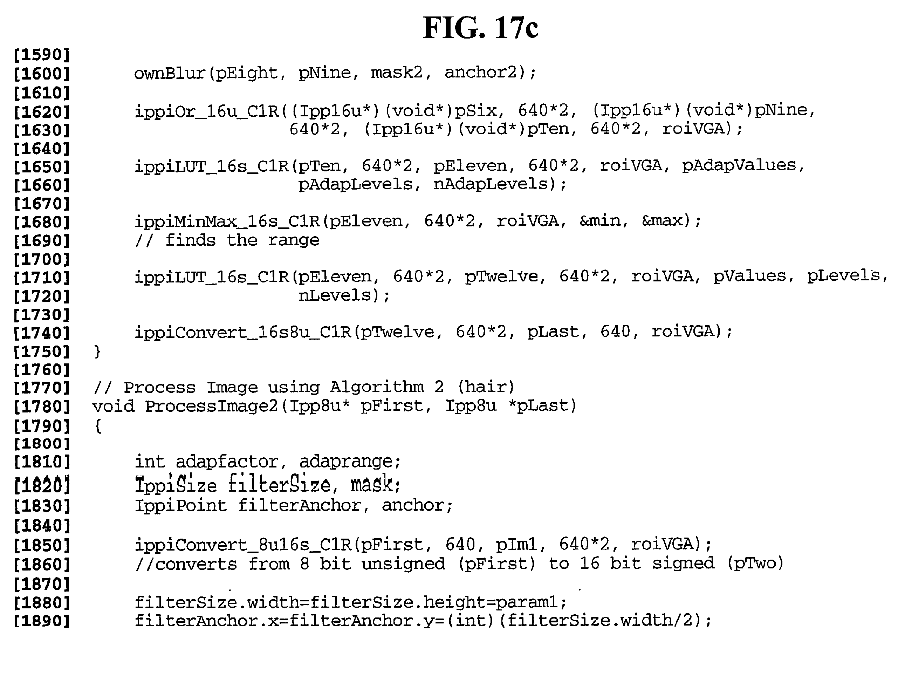

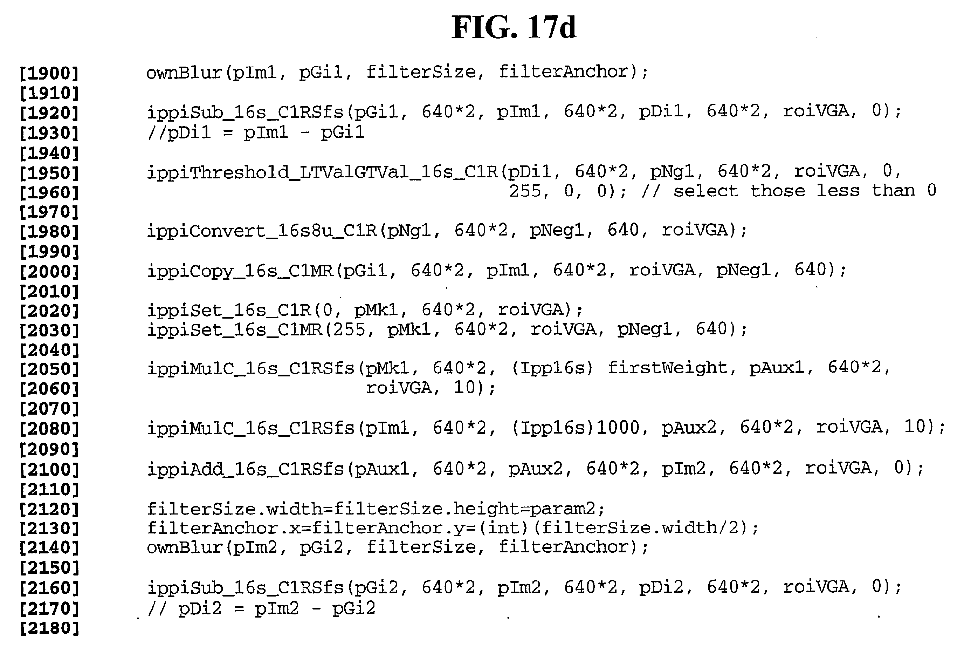

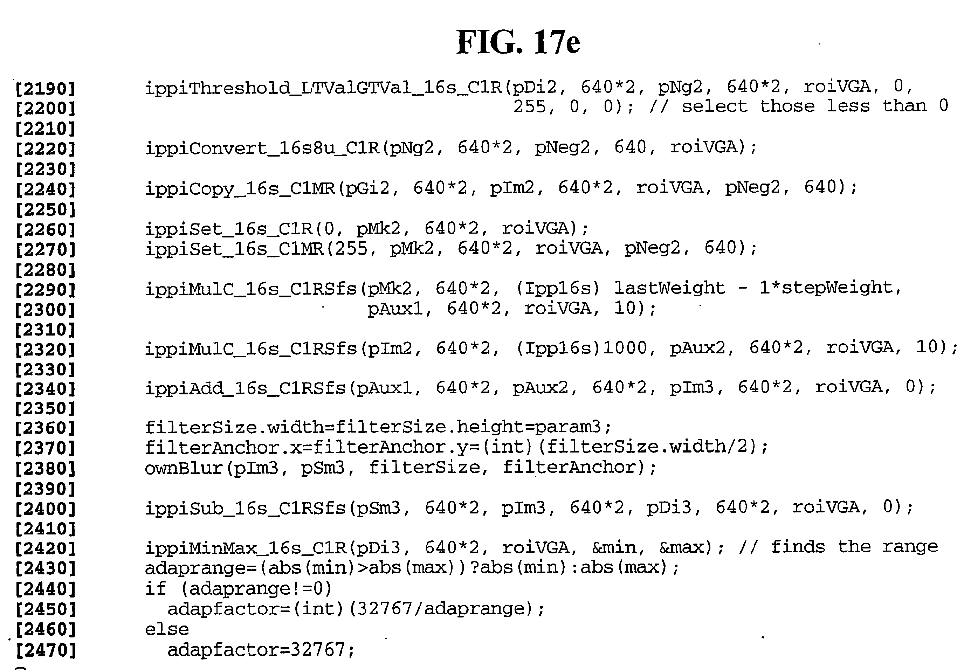

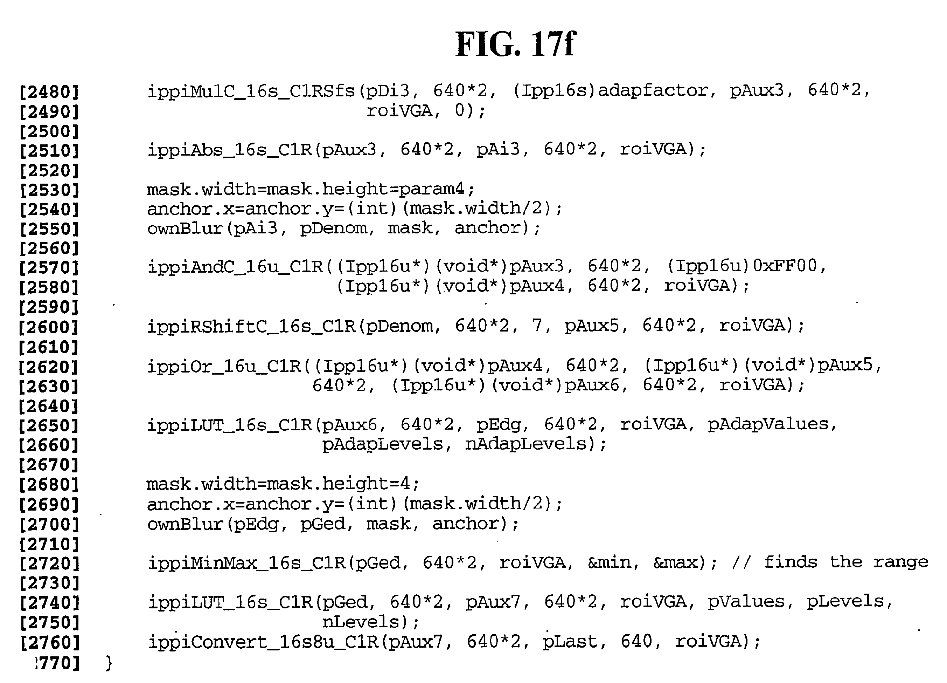

FIGS. 17a, 17b, 17c, 17d, 17e, and 17f, taken together in sequence, are a program listing in the C++ programming language for artifact removal image processing of the received image which is based upon the research/investigation program shown in FIGS. 16a and FIG. 16b, but instead uses the Intel image processing library to perform the mathematical operations more quickly.

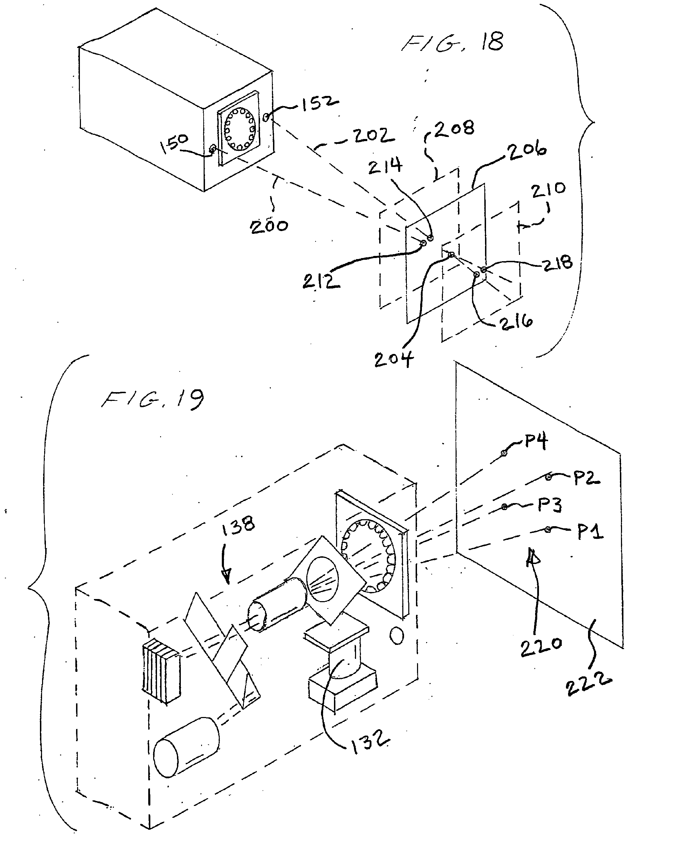

Any or all of the embodiments of the present invention preferably include a mechanism for keeping the image of the buried structure, as seen by the imaging device, in focus to the image device camera with a proper lens-to-subject distance thereto. As seen best in FIG. 18, a first embodiment of this mechanism uses a pair of lasers, **150, 152,** each laser respectively emitting a beam **200, 202,** with beams **200** and **202** being non-parallel with respect to each other and thus being directed toward the object from different angles, such that the two laser beams only converge to the same spot **204** and intersect when the target is at the proper lens-to-subject distance from the imaging device, as shown by the position of intersecting plane **206.** If the target is closer to the apparatus than the proper lens-to-subject distance, as shown by plane **208,** or if the target is further from the apparatus than the proper lens-to-subject distance, as shown by plane **210,** the two laser beams will not intersect at a single point **204** but instead will appear on the surface of the object as a first pair of visible dots **212, 214** (for plane **208**) or as a second pair of visible dots **216, 218** (for plane **210**), indicating that the buried structure is not in focus to the imaging device camera, and that the distance from the object to the apparatus should be changed to bring the viewed image of the buried structure into focus. lasers **150** and **152** may also be seen in FIGS. 12, 13, and 14. Suitable lasers for use with the present invention are the model LM-03 laser modules made by Roithner Lasertechnik, of Vienna, Austria.

A second embodiment of the target positioning mechanism adds a recognizable visible light pattern, such as a text border, independent of the buried structure being observed, to the projected image for mutual projection therewith. The projected recognizable pattern will only be recognized by the human viewer as being in focus on the surface of the target object when the target is at the desired distance from the projector, thereby causing the buried structure beneath the surface of the target to also be at the proper lens-to-subject distance from the imaging device. If desired, cartoon figures appealing to children could be provided as an incentive for children to properly position their body parts for viewing subcutaneous blood vessels, or a hospital's or clinic's logo or name could be used for the pattern. While the projected image of the buried structure is often somewhat blurred from image processing removal of artifacts, humans can quickly tell if a well-known or recognizable visible light pattern is out of focus. An advantage of this second embodiment of the target positioning mechanism, namely, the projection of a recognizable visible light pattern rather than the use of lasers, is that there is a possible hazard of injury, such as blindness, if proper safety precautions are not used with the lasers.

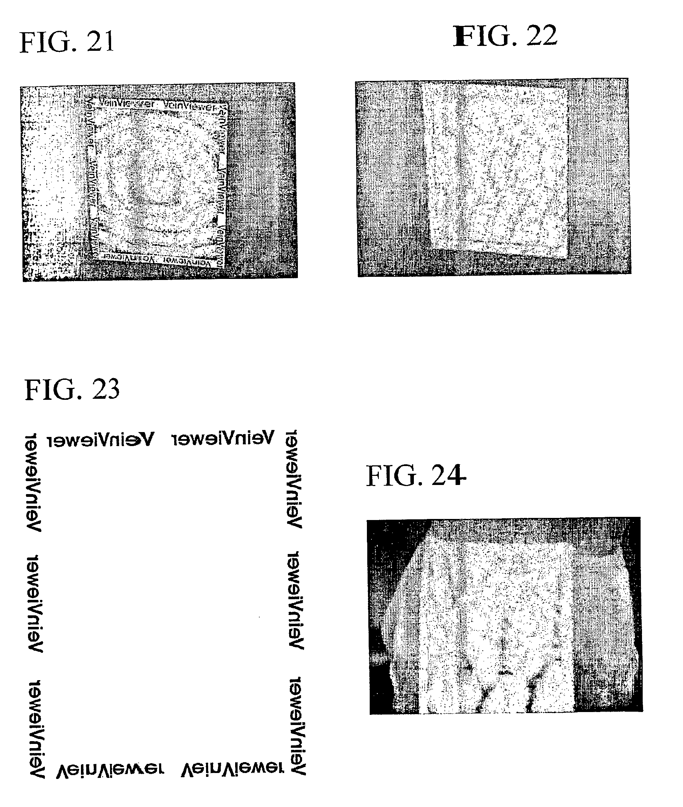

The photograph of FIG. 21 shows a projected image having a text border therearound.

FIG. 22 is another photograph of a projected image having a text border therearound, similar to FIG. 21 but in which the viewed object has been moved out of position, showing how the text border becomes out-of-focus to indicate that the object is not positioned properly with respect to the image device camera.

FIG. 23 shows a text border image that is combined with a projected image for joint projection onto the object to ensure proper positioning. Because of the image reversal that occurs in some embodiments of the invention as images reflect inside the prism structure heretofore described, this text border image is shown reversed but appears unreversed when projected. The projected image is appropriately cropped before combining with the text border so that the text border remains sharp and distinct when projected.

FIG. 24 is a photograph of a processed image of subsurface veins projected onto a hand by the present invention, similar to FIG. 20 (which omits the text border) and FIG. 21 but showing how the text border becomes out of focus to indicate that the hand is not positioned properly.

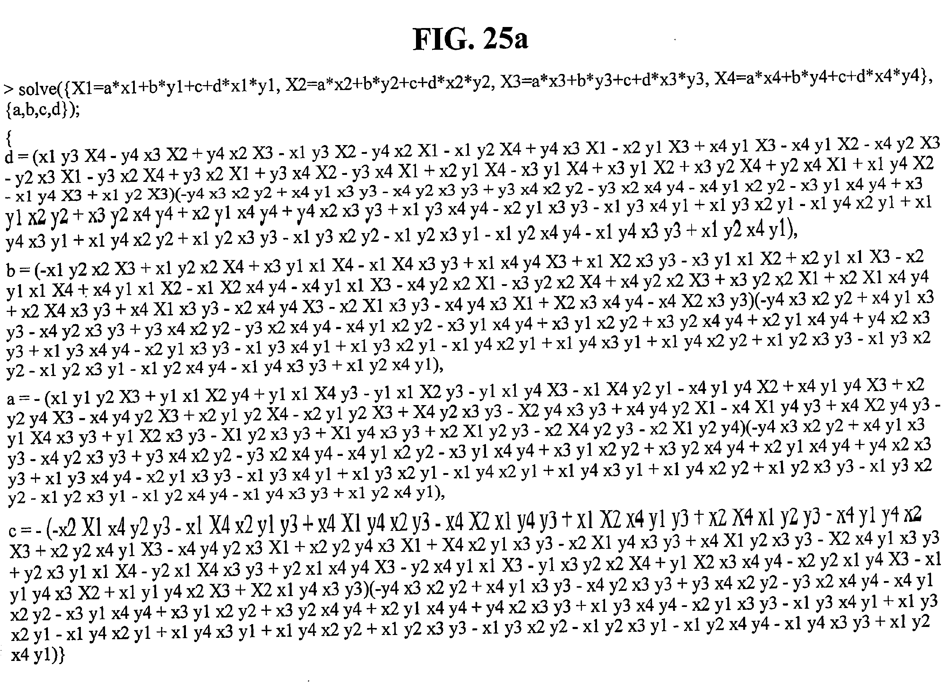

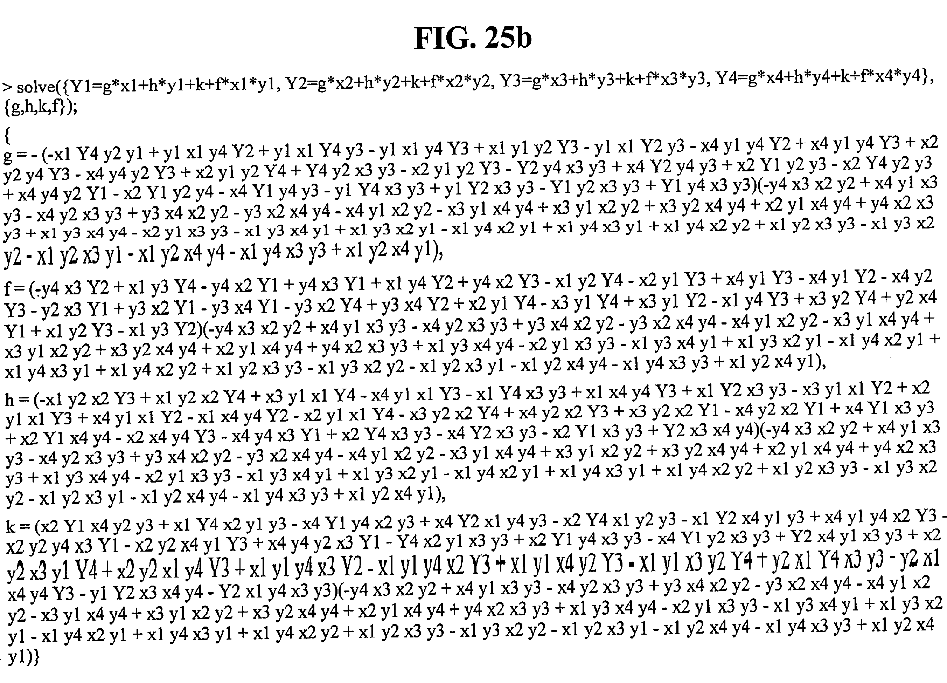

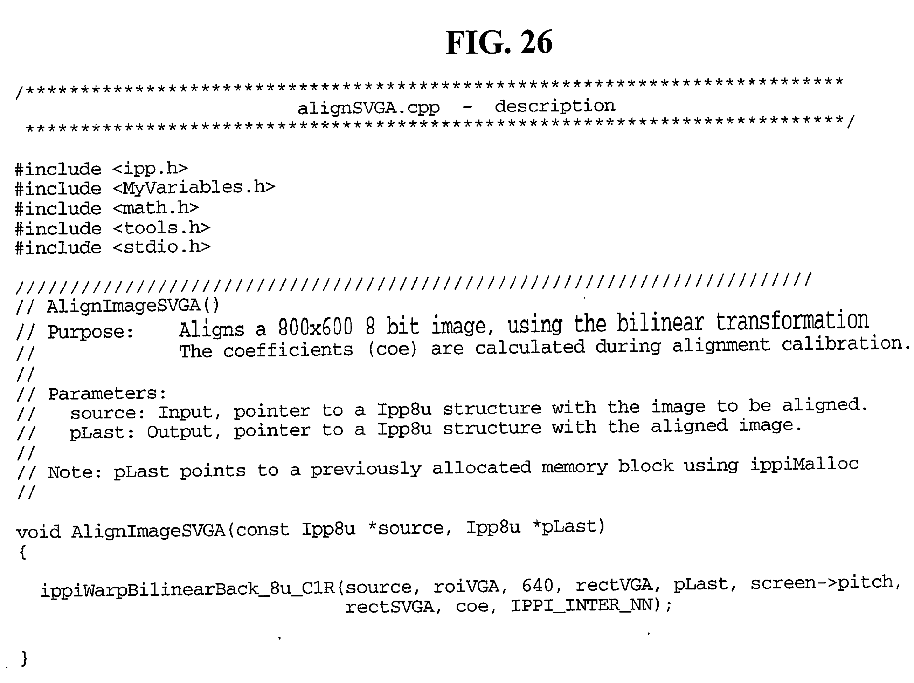

As shown in FIG. 19, a calibration method is provided wherein the video projector **138** (or **162,** or any of the projector of the present invention) projects a green target pattern **220** onto a fluorescent screen **222,** which converts the projected four-dot green target pattern **220** into deep red light that is visible by the infrared imaging device **132.** A computer program records the observed position of the viewed pattern of four projected dots **P1, P2, P3,** and **P4,** in Cartesian coordinates, *i*.*e*., (x1, y1), (x2, y2), (x3, y3), and (x4, y4), versus the desired or "true" position of the dots if alignment were correct, *i*.*e*., (X1, Y1), (X2, Y2), (X3, Y3), and (X4, Y4), and calculates calibration coefficients (a, b, c, d, g, h, k, f) to be used in the bi-linear transformation equations (the arguments to the "solve" function in FIG. 25a and FIG. 25b) to correct magnification, rotation, and translation misalignment between the imaging device and the projector. FIG. 25a and FIG. 25b show the use of the MAPLE 9 computer equation solving program to solve for the bilinear transformation coefficients as a function of the values measured during calibration. These calibration coefficients are used during operation of the device to transform the coordinate system of the image (x, y) into the corrected coordinate system (X, Y) necessary to produce a calibrated image. FIG. 26 shows how these coordinates, once calculated during calibration, are used as parameters to a well-known image processing library mathematical routine provided by the integrated circuit company Intel for use with its processors, to achieve high performance image alignment correction using the bilinear transformation equation. The run-time calculations are done using scaled integer arithmetic, rather than floating point arithmetic, for faster processing of the image.

The calibration procedure projects a test pattern **220,** consisting of four dots **P1, P2, P3,** and **P4,** each having a 25-pixel radius (as viewed by the imaging device camera) at the corners of a rectangle having dimensions of 320 x 240 pixels rectangle (as viewed by the imaging device camera), onto the fluorescent. For example, the camera **132** might have a resolution of 640 x 480 pixels, whereas the projector **138** might have a resolution of 1024 x 780 pixels. Experimental testing for dot radii varying from 4 to 50 pixels showed that the standard deviation of 100 samples decreased rapidly from a dot radius of 5 pixels to about 25 pixels, and then decreased much more slowly out to a radius of 50 pixels.

To practice the calibration method of the present invention, a test pattern of four spaced-apart dots **P1, P2, P3,** and **P4** is projected within a first spectrum, preferably using green light, onto a fluorescent screen **222,** which then fluoresces and produces light within a second spectrum, preferably light adjacent or within the infrared spectrum, such as red light, that is visible to the image device camera **132,** even through the infrared transmitting filter through which the image device camera views its target object. Calibration software then measures the observed position of the four dots and computes the correction coefficients (a, b, c, d, g, f, h, k) for the bi-linear transformation equation, and then uses those coefficients as parameters to the bi-linear transformation in order to correct misalignment errors (rotation, translation, and magnification) between the image device camera and the projector by warping the image prior to projection so that the projected image is corrected for misalignment. It should be noted that this procedure allows for correction of magnification errors that are different in the horizontal and vertical directions, and also allows for correction of translation errors that are different in the horizontal and vertical directions.

Testing has shown that this calibration procedure can correct misalignments as great as +/-25.4 mm to within about half of the image camera's pixel size The alignment is best for image portion near the test pattern's four dots, but remains remarkably good over the entire image.

It should be understood that features of any of these embodiments may be used with another in a way that will now be understood in view of the foregoing disclosure. For example, any embodiment could choose to illuminate the object using infrared components within ambient lighting, rather than providing a separate diffuse infrared light source, and/or could choose between a laser target positioner and a recognizable pattern that is combined with the projected image of the buried structure for maintaining a desired distance from the image device camera to the object.

Although the present invention has been described and illustrated with respect to a preferred embodiment and a preferred use therefor, it is not to be so limited since modifications and changes can be made therein which are within the full intended scope of the invention.

## Claims

1. An apparatus (2, 70, 130, 154, 192) to enhance the visibility of a buried structure beneath the surface of an object, the apparatus comprising:
an imaging device (38, 74) for receiving diffuse light reflected from the object and for producing an image, **characterized in that** it further comprises
a computer (180) for producing a received image and removing small dark artifacts from the received image while retaining larger dark objects in the received image to produce a processed image, and
a first laser (150) having a first emitted beam (200) and a second laser (152) having a second emitted beam (202), said first emitted beam (200) and said second emitted beam (202) being nonparallel with respect to each other and said first and said second emitted beams (200, 202) intersecting at a desired target distance from said apparatus at which, when the intersection of said first and said second emitted beams (200, 202) is upon the surface of the object, said buried structure is in focus to said imaging device.

2. An apparatus (2, 70, 130, 154, 192) according to claim 1, additionally comprising a video projector (50, 138, 162) for projecting a visible light image of the buried structure onto the surface of the object.

3. An apparatus (2, 70, 130, 154, 192) according to claim 1, wherein said apparatus comprising:
an imaging device for receiving diffuse light within a first spectrum reflected from the object and for producing an image, and
a video projector (50, 138, 162) for projecting a visible light image within a second spectrum of the buried structure onto the surface of the object, said first and said second spectrums being substantially non-overlapping.

4. An apparatus (2, 70, 130, 154, 192) according to claims 1 to 3, wherein the apparatus comprising:
video projector means (50, 138, 162) for projecting a visible light image of the buried structure onto the surface of the object, said projected visible light image including a recognizable added pattern that is independent of said buried structure.

5. An apparatus (2, 70, 130, 154, 192) according to claim 4, wherein said added pattern is a text border.

## Patentansprüche

1. Einrichtung (2, 70, 130, 154, 192) zum Verstärken der Sichtbarkeit einer verdeckten Struktur unter der Oberfläche eines Objekts, wobei die Einrichtung aufweist:
eine Abbildungseinrichtung (38, 74) zum Empfangen von diffusem Licht, das von dem Objekt reflektiert worden ist,
und zum Erzeugen eines Bilds, **dadurch gekennzeichnet, dass** sie ferner
einen Computer (180) zum Erzeugen eines empfangenen Bilds und zum Entfernen von kleinen dunklen Artefakten aus dem empfangenen Bild, während größere dunkle Objekte in dem empfangenen Bild beibehalten werden, um ein verarbeitetes Bild zu erzeugen, und
einen ersten Laser (150) mit einem ersten emittierten Strahl (200) und einen zweiten Laser (200) mit einem zweiten emittierten Strahl (202) aufweist, wobei der erste emittierte Strahl (200) und der zweite emittierte Strahl (202) nicht-parallel zueinander sind und der erste und zweite emittierte Strahl (200, 202) sich bei einem vorgegebenen Zielabstand von der Einrichtung schneiden, bei dem, wenn der Schnitt des ersten und zweiten emittierten Strahls (200, 202) auf der Oberfläche des Objekts ist, die verdeckte Struktur im Fokus der Abbildungseinrichtung ist.

2. Einrichtung (2, 70, 130, 154, 192) nach Anspruch 1, die zusätzlich einen Videoprojektor (50, 138, 162) zum Projizieren eines sichtbaren Lichtbilds der verdeckten Struktur auf der Oberfläche des Objekts aufweist.

3. Einrichtung (2, 70, 130, 154, 192) nach Anspruch 1, wobei die Einrichtung aufweist: eine Abbildungseinrichtung zum Empfangen von diffusem Licht in einem ersten Spektrum, das von dem Objekt reflektiert worden ist, und zum Erzeugen eines Bilds, und
einen Videoprojektor (50, 138, 162) zum Projizieren eines sichtbaren Lichtbilds in einem zweiten Spektrum von der verdeckten Struktur auf die Oberfläche des Objekts, wobei das erste und das zweite Spektrum im Wesentlichen nichtüberlappend sind.

4. Einrichtung (2, 70, 130, 154, 192) nach einem der Ansprüche 1 bis 3, wobei die Einrichtung aufweist:
eine Videoprojektoreinrichtung (50, 138, 162) zum Projizieren eines sichtbaren Lichtbilds der verdeckten Struktur auf die Oberfläche des Objekts, wobei das projizierte sichtbare Lichtbild ein wiedererkennbares hinzugefügtes Muster umfasst, das unabhängig von der verdeckten Struktur ist.

5. Einrichtung (2, 70, 130, 154, 192) nach Anspruch 4, wobei das hinzugefügte Muster eine Textgrenze ist.

## Revendications

1. Appareil (2, 70, 130, 154, 192) permettant d'accroître la visibilité d'une structure enterrée au-dessous de la surface d'un objet, l'appareil comportant :
un dispositif de formation d'image (38, 74) pour recevoir une lumière diffuse réfléchie à partir de l'objet et pour produire une image, **caractérisé en ce qu'**il comprend, de plus,
un ordinateur (180) pour produire une image reçue et éliminer de l'image reçue de petits artefacts sombres tout en conservant les objets sombres plus grands dans l'image reçue afin de produire une image traitée, et
un premier laser (150) présentant un premier faisceau émis (200) et un second laser (152) présentant un second faisceau émis (202), ledit premier faisceau émis (200) et ledit second faisceau émis (202) n'étant pas parallèles l'un par rapport à l'autre et ledit premier et ledit second faisceaux émis (200, 202) s'entrecoupant à une distance cible souhaitée à partir dudit appareil au niveau de laquelle, lorsque l'intersection dudit premier et dudit second faisceaux émis (200, 202) se situe sur la surface de l'objet, ladite structure enterrée se trouve focalisée par rapport au dit dispositif de formation d'image.

2. Appareil (2, 70, 130, 154, 192) selon la revendication 1 comprenant, de plus, un projecteur vidéo (50, 138, 162) pour projeter une image en lumière visible de la structure enterrée sur la surface de l'objet.

3. Appareil (2, 70, 130, 154, 192) selon la revendication 1, dans lequel ledit appareil comporté :
un dispositif de formation d'image pour recevoir une lumière diffuse dans les limites d'un premier spectre réfléchi à partir de l'objet et permettant de produire une image, et
un projecteur vidéo (50, 138, 162) pour projeter une image en lumière visible dans les limites d'un second spectre de la structure enterrée sur la surface de l'objet, ledit premier et ledit second spectres étant essentiellement en non chevauchement.

4. Appareil (2, 70, 130, 154,192) selon les revendications 1 à 3, dans lequel l'appareil comporté :
des moyens de projecteur vidéo (50, 138, 162) pour projeter une image en lumière visible de la structure enterrée sur la surface de l'objet, ladite image en lumière visible projetée incluant un motif reconnaissable ajouté qui est indépendant de ladite structure enterrée.

5. Appareil (2, 70, 130, 154, 192) selon la revendication 4, dans lequel ledit motif ajouté est une marge de texte.
